# EUROPEAN PATENT APPLICATION

(11) **EP 2 468 889 A2**
(43) Date of publication of application: **27.06.2012**
(21) Application number: 12151990.4
(22) Date of filing: 08.07.2005
(51) Int. Cl.: C12Q 1/68, C07H 21/02, C12P 19/34, C07H 21/04

(54) **Identification of markers in lung and breast cancer**

(30) Priority: 09.07.2004 US 586599 P; 09.07.2004 US 587019 P
(62) Divisional of application: 05764654.9
(71) Applicant: University of Pittsburgh of the Commonwealth System of Higher Education, Pittsburgh, PA 15260 (US)
(72) Inventor: Godfrey, Tony E., Bronxville, NY 10708 (US); Xi, Liqiang, Plainsboro, NJ 08536 (US); Raja, Siva, Jamaica Plain, MA 02130 (US); Gooding, William E., Pittsburgh, PA 15216 (US)
(74) Representative: Samuels, Lucy Alice

(57) **Abstract**

Methods for identifying expression of markers indicative of the presence of breast cancer and lung cancer are provided. Also provided are articles of manufacture useful in such methods and compositions containing primers and probes useful in such methods.

SEQUENCE LISTING

<110>  University of Pittsburgh - of The Commonwealth System of
       Higher Education
       Godfrey, Tony
       Xi, Liqiang
       Gooding, William E
       Raja, Siva E

<120>  Identification of Markers in Lung and Breast Cancer

<130>  030610

<150>  60/586,599
<151>  2004-07-09

<150>  60/587,019
<151>  2004-07-09

<160>  28

<170>  PatentIn version 3.3

<210>  1
<211>  1753
<212>  DNA
<213>  Homo sapiens

<400>  1
cagccccgcc cctacctgtg gaagcccagc cgcccgctcc cgcggataaa aggtgcggag      60

tgtccccgag gtcagcgagt gcgcgctcct cctcgcccgc cgctaggtcc atcccggccc     120

agccaccatg tccatccact tcagctcccc ggtattcacc tcgcgctcag ccgccttctc     180

gggccgcggc gcccaggtgc gcctgagctc cgctcgcccc ggcggccttg gcagcagcag     240

cctctacggc ctcggcgcct cgcggccgcg cgtggccgtg cgctctgcct atgggggccc     300

ggtgggcgcc ggcatccgcg aggtcaccat taaccagagc ctgctggccc cgctgcggct     360

ggacgccgac ccctccctcc agcgggtgcg ccaggaggag agcgagcaga tcaagaccct     420

caacaacaag tttgcctcct tcatcgacaa ggtgcggttt ctggagcagc agaacaagct     480

gctggagacc aagtggacgc tgctgcagga gcagaagtcg gccaagagca gccgcctccc     540

agacatcttt gaggcccaga ttgctggcct tcggggtcag cttgaggcac tgcaggtgga     600

tgggggccgc ctggaggcgg agctgcggag catgcaggat gtggtggagg acttcaagaa     660

taagtacgaa gatgaaatta accgccgcac agctgctgag aatgagtttg tggtgctgaa     720

gaaggatgtg gatgctgcct acatgagcaa ggtggagctg gaggccaagg tggatgccct     780

gaatgatgag atcaacttcc tcaggaccct caatgagacg gagttgacag agctgcagtc     840

ccagatctcc gacacatctg tggtgctgtc catggacaac agtcgctccc tggacctgga     900

cggcatcatc gctgaggtca aggcacagta tgaggagatg gccaaatgca gccgggctga     960

ggctgaagcc tggtaccaga ccaagtttga gaccctccag gcccaggctg ggaagcatgg    1020

ggacgacctc cggaataccc ggaatgagat ttcagagatg aaccgggcca tccagaggct    1080

gcaggctgag atcgacaaca tcaagaacca gcgtgccaag ttggaggccg ccattgccga 1140

ggctgaggag cgtggggagc tggcgctcaa ggatgctcgt gccaagcagg aggagctgga 1200

agccgccctg cagcgggcca agcaggatat ggcacggcag ctgcgtgagt accaggaact 1260

catgagcgtg aagctggccc tggacatcga gatcgccacc taccgcaagc tgctggaggg 1320

cgaggagagc cggttggctg gagatggagt gggagccgtg aatatctctg tgatgaattc 1380

cactggtggc agtagcagtg gcggtggcat tgggctgacc ctcgggggaa ccatgggcag 1440

caatgccctg agcttctcca gcagtgcggg tcctgggctc ctgaaggctt attccatccg 1500

gaccgcatcc gccagtcgca ggagtgcccg cgactgagcc gcctcccacc actccactcc 1560

tccagccacc acccacaatc acaagaagat tcccacccct gcctcccatg cctggtccca 1620

agacagtgag acagtctgga aagtgatgtc agaatagctt ccaataaagc agcctcattc 1680

tgaggcctga gtgatccacg tgaaaaaaaa aaaaaaaaaa aaaaaaaaaa aaaaaaaaaa 1740

aaaaaaaaaa aaa 1753

<210> 2
<211> 1440
<212> DNA
<213> Homo sapiens

<400> 2
cgcccctgac accattcctc ccttcccccc tccaccggcc gcgggcataa aaggcgccag 60

gtgagggcct cgccgctcct cccgcgaatc gcagcttctg agaccagggt tgctccgtcc 120

gtgctccgcc tcgccatgac ttcctacagc tatcgccagt cgtcggccac gtcgtccttc 180

ggaggcctgg gcggcggctc cgtgcgtttt gggccggggg tcgcctttcg cgcgcccagc 240

attcacgggg gctccggcgg ccgcggcgta tccgtgtcct ccgcccgctt tgtgtcctcg 300

tcctcctcgg gggcctacgg cggcggctac ggcggcgtcc tgaccgcgtc cgacgggctg 360

ctggcgggca acgagaagct aaccatgcag aacctcaacg accgcctggc ctcctacctg 420

gacaaggtgc gcgccctgga ggcggccaac ggcgagctag aggtgaagat ccgcgactgg 480

taccagaagc aggggcctgg gccctcccgc gactacagcc actactacac gaccatccag 540

gacctgcggg acaagattct tggtgccacc attgagaact ccaggattgt cctgcagatc 600

gacaatgccc gtctggctgc agatgacttc cgaaccaagt ttgagacgga acaggctctg 660

cgcatgagcg tggaggccga catcaacggc ctgcgcaggg tgctggatga gctgaccctg 720

gccaggaccg acctggagat gcagatcgaa ggcctgaagg aagagctggc ctacctgaag 780

aagaaccatg aggaggaaat cagtacgctg aggggccaag tgggaggcca ggtcagtgtg 840

gaggtggatt ccgctccggg caccgatctc gccaagatcc tgagtgacat gcgaagccaa 900

tatgaggtca tggccgagca gaaccggaag gatgctgaag cctggttcac cagccggact 960

gaagaattga accgggaggt cgctggccac acggagcagc tccagatgag caggtccgag 1020

gttactgacc tgcggcgcac ccttcagggt cttgagattg agctgcagtc acagctgagc 1080

atgaaagctg ccttggaaga cacactggca gaaacggagg cgcgctttgg agcccagctg 1140

gcgcatatcc aggcgctgat cagcggtatt gaagcccagc tgggcgatgt gcgagctgat 1200

agtgagcggc agaatcagga gtaccagcgg ctcatggaca tcaagtcgcg gctggagcag 1260

gagattgcca cctaccgcag cctgctcgag ggacaggaag atcactacaa caatttgtct 1320

gcctccaagg tcctctgagg cagcaggctc tggggcttct gctgtccttt ggagggtgtc 1380

ttctgggtag agggatggga aggaagggac ccttaccccc ggctcttctc ctgacctgcc 1440

<210> 3
<211> 503
<212> DNA
<213> Homo sapiens

<400> 3
gacagcggct tccttgatcc ttgccacccg cgactgaaca ccgacagcag cagcctcacc 60

atgaagttgc tgatggtcct catgctggcg gccctctccc agcactgcta cgcaggctct 120

ggctgcccct tattggagaa tgtgatttcc aagacaatca atccacaagt gtctaagact 180

gaatacaaag aacttcttca agagttcata gacgacaatg ccactacaaa tgccatagat 240

gaattgaagg aatgttttct taaccaaacg gatgaaactc tgagcaatgt tgaggtgttt 300

atgcaattaa tatatgacag cagtctttgt gatttatttt aactttctgc aagacctttg 360

gctcacagaa ctgcagggta tggtgagaaa ccaactacgg attgctgcaa accacacctt 420

ctctttctta tgtcttttta ctacaaacta caagacaatt gttgaaacct gctatacatg 480

tttattttaa taaattgatg gca 503

<210> 4
<211> 517
<212> DNA
<213> Homo sapiens

<400> 4
cctccacagc aacttccttg atccctgcca cgcacgactg aacacagaca gcagccgcct 60

cgccatgaag ctgctgatgg tcctcatgct ggcggccctc ctcctgcact gctatgcaga 120

ttctggctgc aaactcctgg aggacatggt tgaaaagacc atcaattccg acatatctat 180

acctgaatac aaagagcttc ttcaagagtt catagacagt gatgccgctg cagaggctat 240

ggggaaattc aagcagtgtt tcctcaacca gtcacataga actctgaaaa actttggact 300

gatgatgcat acagtgtacg acagcatttg gtgtaatatg aagagtaatt aactttaccc 360

aaggcgtttg gctcagaggg ctacagacta tggccagaac tcatctgttg attgctagaa 420

accacttttc tttcttgtgt tgtcttttta tgtggaaact gctagacaac tgttgaaacc 480

tcaaattcat ttccatttca ataactaact gcaaatc 517

<210> 5
<211> 576
<212> DNA
<213> Homo sapiens

<400> 5
cttctctggg acacattgcc ttctgttttc tccagcatgc gcttgctcca gctcctgttc 60

agggccagcc ctgccaccct gctcctggtt ctctgcctgc agttgggggc caacaaagct 120

caggacaaca ctcggaagat cataataaag aattttgaca ttcccaagtc agtacgtcca 180

aatgacgaag tcactgcagt gcttgcagtt caaacagaat tgaaagaatg catggtggtt 240

aaaacttacc tcattagcag catccctcta caaggtgcat ttaactataa gtatactgcc 300

tgcctatgtg acgacaatcc aaaaaccttc tactgggact tttacaccaa cagaactgtg 360

caaattgcag ccgtcgttga tgttattcgg gaattaggca tctgccctga tgatgctgct 420

gtaatcccca tcaaaaacaa ccggttttat actattgaaa tcctaaaggt agaataatgg 480

aagccctgtc tgtttgccac acccaggtga tttcctctaa agaaacttgg ctggaatttc 540

tgctgtggtc tataaaataa acttcttaac atgctt 576

<210> 6
<211> 3336
<212> DNA
<213> Homo sapiens

<400> 6
ttttcttaga cattaactgc agacggctgg caggatagaa gcagcggctc acttggactt 60

tttcaccagg gaaatcagag acaatgatgg ggctcttccc cagaactaca ggggctctgg 120

ccatcttcgt ggtggtcata ttggttcatg gagaattgcg aatagagact aaaggtcaat 180

atgatgaaga agagatgact atgcaacaag ctaaaagaag gcaaaaacgt gaatgggtga 240

aatttgccaa accctgcaga gaaggagaag ataactcaaa aagaaaccca attgccaaga 300

ttacttcaga ttaccaagca acccagaaaa tcacctaccg aatctctgga gtgggaatcg 360

atcagccgcc ttttggaatc tttgttgttg acaaaaacac tggagatatt aacataacag 420

ctatagtcga ccgggaggaa actccaagct tcctgatcac atgtcgggct ctaaatgccc 480

aaggactaga tgtagagaaa ccacttatac taacggttaa aattttggat attaatgata 540

atcctccagt attttcacaa caaattttca tgggtgaaat tgaagaaaat agtgcctcaa 600

actcactggt gatgatacta aatgccacag atgcagatga accaaaccac ttgaattcta 660

aaattgcctt caaaattgtc tctcaggaac cagcaggcac acccatgttc ctcctaagca 720

gaaacactgg ggaagtccgt actttgacca attctcttga ccgagagcaa gctagcagct 780

atcgtctggt tgtgagtggt gcagacaaag atggagaagg actatcaact caatgtgaat 840

gtaatattaa agtgaaagat gtcaacgata acttcccaat gtttagagac tctcagtatt 900

cagcacgtat tgaagaaaat attttaagtt ctgaattact tcgatttcaa gtaacagatt 960

tggatgaaga gtacacagat aattggcttg cagtatattt ctttacctct gggaatgaag 1020

gaaattggtt tgaaatacaa actgatccta gaactaatga aggcatcctg aaagtggtga 1080

aggctctaga ttatgaacaa ctacaaagcg tgaaacttag tattgctgtc aaaaacaaag 1140

ctgaatttca ccaatcagtt atctctcgat accgagttca gtcaacccca gtcacaattc 1200

aggtaataaa tgtaagagaa ggaattgcat tccgtcctgc ttccaagaca tttactgtgc 1260

aaaaaggcat aagtagcaaa aaattggtgg attatatcct gggaacatat caagccatcg 1320

atgaggacac taacaaagct gcctcaaatg tcaaatatgt catgggacgt aacgatggtg 1380

gatacctaat gattgattca aaaactgctg aaatcaaatt tgtcaaaaat atgaaccgag 1440

attctacttt catagttaac aaaacaatca cagctgaggt tctggccata gatgaataca 1500

cgggtaaaac ttctacaggc acggtatatg ttagagtacc cgatttcaat gacaattgtc 1560

caacagctgt cctcgaaaaa gatgcagttt gcagttcttc accttccgtg gttgtctccg 1620

ctagaacact gaataataga tacactggcc cctatacatt tgcactggaa gatcaacctg 1680

taaagttgcc tgccgtatgg agtatcacaa ccctcaatgc tacctcggcc ctcctcagag 1740

cccaggaaca gatacctcct ggagtatacc acatctccct ggtacttaca gacagtcaga 1800

acaatcggtg tgagatgcca cgcagcttga cactggaagt ctgtcagtgt gacaacaggg 1860

gcatctgtgg aacttcttac ccaaccacaa gccctgggac caggtatggc aggccgcact 1920

cagggaggct ggggcctgcc gccatcggcc tgctgctcct tggtctcctg ctgctgctgt 1980

tggcccccct tctgctgttg acctgtgact gtggggcagg ttctactggg ggagtgacag 2040

gtggttttat cccagttcct gatggctcag aaggaacaat tcatcagtgg ggaattgaag 2100

gagcccatcc tgaagacaag gaaatcacaa atatttgtgt gcctcctgta acagccaatg 2160

gagccgattt catggaaagt tctgaagttt gtacaaatac gtatgccaga ggcacagcgg 2220

tggaaggcac ttcaggaatg gaaatgacca ctaagcttgg agcagccact gaatctggag 2280

gtgctgcagg ctttgcaaca gggacagtgt caggagctgc ttcaggattc ggagcagcca 2340

ctggagttgg catctgttcc tcagggcagt ctggaaccat gagaacaagg cattccactg 2400

gaggaaccaa taaggactac gctgatgggg cgataagcat gaattttctg gactcctact 2460

tttctcagaa agcatttgcc tgtgcggagg aagacgatgg ccaggaagca aatgactgct 2520

tgttgatcta tgataatgaa ggcgcagatg ccactggttc tcctgtgggc tccgtgggtt 2580

gttgcagttt tattgctgat gacctggatg acagcttctt ggactcactt ggacccaaat 2640

ttaaaaaact tgcagagata agccttggtg ttgatggtga aggcaaagaa gttcagccac 2700

cctctaaaga cagcggttat gggattgaat cctgtggcca tcccatagaa gtccagcaga 2760

caggatttgt taagtgccag actttgtcag gaagtcaagg agcttctgct ttgtccgcct 2820

ctgggtctgt ccagccagct gtttccatcc ctgaccctct gcagcatggt aactatttag 2880

taacggagac ttactcggct tctggttccc tcgtgcaacc ttccactgca ggctttgatc 2940

cacttctcac acaaaatgtg atagtgacag aaagggtgat ctgtcccatt tccagtgttc 3000

ctggcaacct agctggccca acgcagctac gagggtcaca tactatgctc tgtacagagg 3060

atccttgctc ccgtctaata tgaccagaat gagctggaat accacactga ccaaatctgg 3120

atctttggac taaagtattc aaaatagcat agcaaagctc actgtattgg gctaataatt 3180

tggcacttat tagcttctct cataaactga tcacgattat aaattaaatg tttgggttca 3240

taccccaaaa gcaatatgtt gtcactccta attctcaagt actattcaaa ttgtagtaaa 3300

tcttaaagtt tttcaaaacc ctaaaatcat attcgc 3336

<210> 7
<211> 1694
<212> DNA
<213> Homo sapiens

<400> 7
ctctctgccc acctctgctt cctctaggaa cacaggagtt ccagatcaca tcgagttcac 60

catgaattca ctcagtgaag ccaacaccaa gttcatgttc gacctgttcc aacagttcag 120

aaaatcaaaa gagaacaaca tcttctattc ccctatcagc atcacatcag cattagggat 180

ggtcctctta ggagccaaag acaacactgc acaacagatt aagaaggttc ttcactttga 240

tcaagtcaca gagaacacca caggaaaagc tgcaacatat catgttgata ggtcaggaaa 300

tgttcatcac cagtttcaaa agcttctgac tgaattcaac aaatccactg atgcatatga 360

gctgaagatc gccaacaagc tcttcggaga aaaaacgtat ctatttttac aggaatattt 420

agatgccatc aagaaatttt accagaccag tgtggaatct gttgattttg caaatgctcc 480

agaagaaagt cgaaagaaga ttaactcctg ggtggaaagt caaacgaatg aaaaaattaa 540

aaacctaatt cctgaaggta atattggcag caataccaca ttggttcttg tgaacgcaat 600

ctatttcaaa gggcagtggg agaagaaatt taataaagaa gatactaaag aggaaaaatt 660

ttggccaaac aagaatacat acaagtccat acagatgatg aggcaataca catcttttca 720

ttttgcctcg ctggaggatg tacaggccaa ggtcctggaa ataccataca aaggcaaaga 780

tctaagcatg attgtgttgc tgccaaatga aatcgatggt ctccagaagc ttgaagagaa 840

actcactgct gagaaattga tggaatggac aagtttgcag aatatgagag agacacgtgt 900

cgatttacac ttacctcggt tcaaagtgga agagagctat gacctcaagg acacgttgag 960

aaccatggga atggtggata tcttcaatgg ggatgcagac ctctcaggca tgaccgggag 1020

ccgcggtctc gtgctatctg gagtcctaca caaggccttt gtggaggtta cagaggaggg 1080

agcagaagct gcagctgcca ccgctgtagt aggattcgga tcatcaccta cttcaactaa 1140

tgaagagttc cattgtaatc accctttcct attcttcata aggcaaaata agaccaacag 1200

catcctcttc tatggcagat tctcatcccc gtagatgcaa ttagtctgtc actccatttg 1260

gaaaatgttc acctgcagat gttctggtaa actgattgct ggcaacaaca gattctcttg 1320

gctcatattt cttttctttc tcatcttgat gatgatcgtc atcatcaaga atttaatgat 1380

taaaatagca tgcctttctc tctttctctt aataagccca catataaatg tactttttct 1440

tccagaaaaa ttctccttga ggaaaaatgt ccaaaataag atgaatcact taataccgta 1500

tcttctaaat ttgaaatata attctgtttg tgacctgttt taaatgaacc aaaccaaatc 1560

atactttttc tttgaattta gcaacctaga aacacacatt tctttgaatt taggtgatac 1620

ctaaatcctt cttatgtttc taaattttgt gattctataa aacacatcat caataaaata 1680

gtgacataaa atca 1694

<210> 8
<211> 1782
<212> DNA
<213> Homo sapiens

<400> 8
gggagacaca cacagcctct ctgcccacct ctgcttcctc taggaacaca ggagttccag 60

atcacatcga gttcaccatg aattcactca gtgaagccaa caccaagttc atgttcgatc 120

tgttccaaca gttcagaaaa tcaaaagaga acaacatctt ctattcccct atcagcatca 180

catcagcatt agggatggtc ctcttaggag ccaaagacaa cactgcacaa caaattagca 240

aggttcttca ctttgatcaa gtcacagaga acaccacaga aaaagctgca acatatcatg 300

ttgataggtc aggaaatgtt catcaccagt ttcaaaagct tctgactgaa ttcaacaaat 360

ccactgatgc atatgagctg aagatcgcca acaagctctt cggagaaaag acgtatcaat 420

ttttacagga atatttagat gccatcaaga aattttacca gaccagtgtg gaatctactg 480

attttgcaaa tgctccagaa gaaagtcgaa agaagattaa ctcctgggtg gaaagtcaaa 540

cgaatgaaaa aattaaaaac ctatttcctg atgggactat tggcaatgat acgacactgg 600

ttcttgtgaa cgcaatctat ttcaaagggc agtgggagaa taaatttaaa aaagaaaaca 660

ctaaagagga aaaattttgg ccaaacaaga atacatacaa atctgtacag atgatgaggc 720

aatacaattc ctttaatttt gccttgctgg aggatgtaca ggccaaggtc ctggaaatac 780

catacaaagg caaagatcta agcatgattg tgctgctgcc aaatgaaatc gatggtctgc 840

agaagcttga agagaaactc actgctgaga aattgatgga atggacaagt ttgcagaata 900

tgagagagac atgtgtcgat ttacacttac ctcggttcaa aatggaagag agctatgacc 960

tcaaggacac gttgagaacc atgggaatgg tgaatatctt caatggggat gcagacctct 1020

caggcatgac ctggagccac ggtctctcag tatctaaagt cctacacaag gcctttgtgg 1080

aggtcactga ggagggagtg gaagctgcag ctgccaccgc tgtagtagta gtcgaattat 1140

catctccttc aactaatgaa gagttctgtt gtaatcaccc tttcctattc ttcataaggc 1200

aaaataagac caacagcatc ctcttctatg gcagattctc atccccatag atgcaattag 1260

tctgtcactc catttagaaa atgttcacct agaggtgttc tggtaaactg attgctggca 1320

acaacagatt ctcttggctc atatttcttt tctatctcat cttgatgatg atagtcatca 1380

tcaagaattt aatgattaaa atagcatgcc tttctctctt tctcttaata agcccacata 1440

taaatgtact tttccttcca gaaaaatttc ccttgaggaa aaatgtccaa gataagatga 1500

atcatttaat accgtgtctt ctaaatttga aatataattc tgtttctgac ctgttttaaa 1560

tgaaccaaac caaatcatac tttctcttca aatttagcaa cctagaaaca cacatttctt 1620

tgaatttagg tgatacctaa atccttctta tgtttctaaa ttttgtgatt ctataaaaca 1680

catcatcaat aaaataatga ctaaaaaaaa aaaaaaaaaa aaaaaaaaaa aaaaaaaaaa 1740

aaaaaaaaaa aacccaaaaa aaaaaaaaaa aaaaaaaaaa aa 1782

<210> 9
<211> 1146
<212> DNA
<213> Homo sapiens

<400> 9
atggctgagt cacacctgct gcagtggctg ctgctgctgc tgcccacgct ctgtggccca 60

ggcactgctg cctggaccac ctcatccttg gcctgtgccc agggccctga gttctggtgc 120

caaagcctgg agcaagcatt gcagtgcaga gccctagggc attgcctaca ggaagtctgg 180

ggacatgtgg gagccgatga cctatgccaa gagtgtgagg acatcgtcca catccttaac 240

aagatggcca aggaggccat tttccaggac acgatgagga agttcctgga gcaggagtgc 300

aacgtcctcc ccttgaagct gctcatgccc cagtgcaacc aagtgcttga cgactacttc 360

cccctggtca tcgactactt ccagaaccag attgactcaa acggcatctg tatgcacctg 420

ggcctgtgca aatcccggca gccagagcca gagcaggagc cagggatgtc agaccccctg 480

cccaaacctc tgcgggaccc tctgccagac cctctgctgg acaagctcgt cctccctgtg 540

ctgcccgggg ccctccaggc gaggcctggg cctcacacac aggatctctc cgagcagcaa 600

ttccccattc ctctccccta ttgctggctc tgcagggctc tgatcaagcg gatccaagcc 660

atgattccca agggtgcgct acgtgtggca gtggcccagg tgtgccgcgt ggtacctctg 720

gtggcgggcg gcatctgcca gtgcctggct gagcgctact ccgtcatcct gctcgacacg 780

ctgctgggcc gcatgctgcc ccagctggtc tgccgcctcg tcctccggtg ctccatggat 840

gacagcgctg gcccaaggtc gccgacagga gaatggctgc cgcgagactc tgagtgccac 900

ctctgcatgt ccgtgaccac ccaggccggg aacagcagcg agcaggccat accacaggca 960

atgctccagg cctgtgttgg ctcctggctg gacaggaaaa agtgcaagca atttgtggag 1020

cagcacacgc cccagctgct gaccctggtg cccaggggct gggatgccca caccacctgc 1080

caggccctcg gggtgtgtgg gaccatgtcc agccctctcc agtgtatcca cagccccgac 1140

ctttga 1146

<210>  10
<211>  1528
<212>  DNA
<213>  Homo sapiens

<400>  10
cggcgagcga gcaccttcga cgcggtccgg ggaccccctc gtcgctgtcc tcccgacgcg      60

gacccgcgtg ccccaggcct cgcgctgccc ggccggctcc tcgtgtccca ctcccggcgc     120

acgccctccc gcgagtcccg ggcccctccc gcgcccctct tctcggcgcg cgcgcagcat     180

ggcgcccccg caggtcctcg cgttcgggct tctgcttgcc gcggcgacgg cgacttttgc     240

cgcagctcag gaagaatgtg tctgtgaaaa ctacaagctg gccgtaaact gctttgtgaa     300

taataatcgt caatgccagt gtacttcagt tggtgcacaa aatactgtca tttgctcaaa     360

gctggctgcc aaatgtttgg tgatgaaggc agaaatgaat ggctcaaaac ttgggagaag     420

agcaaaacct gaaggggccc tccagaacaa tgatgggctt tatgatcctg actgcgatga     480

gagcgggctc tttaaggcca agcagtgcaa cggcacctcc acgtgctggt gtgtgaacac     540

tgctggggtc agaagaacag acaaggacac tgaaataacc tgctctgagc gagtgagaac     600

ctactggatc atcattgaac taaaacacaa agcaagagaa aaaccttatg atagtaaaag     660

tttgcggact gcacttcaga aggagatcac aacgcgttat caactggatc caaaatttat     720

cacgagtatt ttgtatgaga ataatgttat cactattgat ctggttcaaa attcttctca     780

aaaaactcag aatgatgtgg acatagctga tgtggcttat tattttgaaa aagatgttaa     840

aggtgaatcc ttgtttcatt ctaagaaaat ggacctgaca gtaaatgggg aacaactgga     900

tctggatcct ggtcaaactt taatttatta tgttgatgaa aaagcacctg aattctcaat     960

gcagggtcta aaagctggtg ttattgctgt tattgtggtt gtggtgatag cagttgttgc    1020

tggaattgtt gtgctggtta tttccagaaa gaagagaatg gcaaagtatg agaaggctga    1080

gataaaggag atgggtgaga tgcataggga actcaatgca taactatata atttgaagat    1140

tatagaagaa gggaaatagc aaatggacac aaattacaaa tgtgtgtgcg tgggacgaag    1200

acatctttga aggtcatgag tttgttagtt taacatcata tatttgtaat agtgaaacct    1260

gtactcaaaa tataagcagc ttgaaactgg ctttaccaat cttgaaattt gaccacaagt    1320

gtcttatata tgcagatcta atgtaaaatc cagaacttgg actccatcgt taaaattatt    1380

tatgtgtaac attcaaatgt gtgcattaaa tatgcttcca cagtaaaatc tgaaaaactg    1440

atttgtgatt gaaagctgcc tttctattta cttgagtctt gtacatacat acttttttat     1500

gagctatgaa ataaaacatt ttaaactg                                        1528


<210>  11
<211>  2974
<212>  DNA
<213>  Homo sapiens

<400>  11

ctcagggcag agggaggaag gacagcagac cagacagtca cagcagcctt gacaaaacgt 60

tcctggaact caagctcttc tccacagagg aggacagagc agacagcaga gaccatggag 120

tctccctcgg cccctcccca cagatggtgc atcccctggc agaggctcct gctcacagcc 180

tcacttctaa ccttctggaa cccgcccacc actgccaagc tcactattga atccacgccg 240

ttcaatgtcg cagaggggaa ggaggtgctt ctacttgtcc acaatctgcc ccagcatctt 300

tttggctaca gctggtacaa aggtgaaaga gtggatggca accgtcaaat tataggatat 360

gtaataggaa ctcaacaagc taccccaggg cccgcataca gtggtcgaga gataatatac 420

cccaatgcat ccctgctgat ccagaacatc atccagaatg acacaggatt ctacacccta 480

cacgtcataa agtcagatct tgtgaatgaa gaagcaactg gccagttccg ggtatacccg 540

gagctgccca agccctccat ctccagcaac aactccaaac ccgtggagga caaggatgct 600

gtggccttca cctgtgaacc tgagactcag gacgcaacct acctgtggtg ggtaaacaat 660

cagagcctcc cggtcagtcc caggctgcag ctgtccaatg gcaacaggac cctcactcta 720

ttcaatgtca caagaaatga cacagcaagc tacaaatgtg aaacccagaa cccagtgagt 780

gccaggcgca gtgattcagt catcctgaat gtcctctatg gcccggatgc ccccaccatt 840

tcccctctaa acacatctta cagatcaggg gaaaatctga acctctcctg ccacgcagcc 900

tctaacccac ctgcacagta ctcttggttt gtcaatggga ctttccagca atccacccaa 960

gagctcttta tccccaacat cactgtgaat aatagtggat cctatacgtg ccaagcccat 1020

aactcagaca ctggcctcaa taggaccaca gtcacgacga tcacagtcta tgcagagcca 1080

cccaaaccct tcatcaccag caacaactcc aaccccgtgg aggatgagga tgctgtagcc 1140

ttaacctgtg aacctgagat tcagaacaca acctacctgt ggtgggtaaa taatcagagc 1200

ctcccggtca gtcccaggct gcagctgtcc aatgacaaca ggaccctcac tctactcagt 1260

gtcacaagga atgatgtagg accctatgag tgtggaatcc agaacgaatt aagtgttgac 1320

cacagcgacc cagtcatcct gaatgtcctc tatggcccag acgaccccac catttccccc 1380

tcatacacct attaccgtcc aggggtgaac ctcagcctct cctgccatgc agcctctaac 1440

ccacctgcac agtattcttg gctgattgat gggaacatcc agcaacacac acaagagctc 1500

tttatctcca acatcactga gaagaacagc ggactctata cctgccaggc caataactca 1560

gccagtggcc acagcaggac tacagtcaag acaatcacag tctctgcgga gctgcccaag 1620

ccctccatct ccagcaacaa ctccaaaccc gtggaggaca aggatgctgt ggccttcacc 1680

tgtgaacctg aggctcagaa cacaacctac ctgtggtggg taaatggtca gagcctccca 1740

gtcagtccca ggctgcagct gtccaatggc aacaggaccc tcactctatt caatgtcaca 1800

agaaatgacg caagagccta tgtatgtgga atccagaact cagtgagtgc aaaccgcagt 1860

gacccagtca ccctggatgt cctctatggg ccggacaccc ccatcatttc ccccccagac 1920

tcgtcttacc tttcgggagc gaacctcaac ctctcctgcc actcggcctc taacccatcc 1980

ccgcagtatt cttggcgtat caatgggata ccgcagcaac acacacaagt tctctttatc 2040

gccaaaatca cgccaaataa taacgggacc tatgcctgtt ttgtctctaa cttggctact 2100

ggccgcaata attccatagt caagagcatc acagtctctg catctggaac ttctcctggt 2160

ctctcagctg gggccactgt cggcatcatg attggagtgc tggttggggt tgctctgata 2220

tagcagccct ggtgtagttt cttcatttca ggaagactga cagttgtttt gcttcttcct 2280

taaagcattt gcaacagcta cagtctaaaa ttgcttcttt accaaggata tttacagaaa 2340

agactctgac cagagatcga gaccatccta gccaacatcg tgaaacccca tctctactaa 2400

aaatacaaaa atgagctggg cttggtggcg cgcacctgta gtcccagtta ctcgggaggc 2460

tgaggcagga gaatcgcttg aacccgggag gtggagattg cagtgagccc agatcgcacc 2520

actgcactcc agtctggcaa cagagcaaga ctccatctca aaaagaaaag aaaagaagac 2580

tctgacctgt actcttgaat acaagtttct gataccactg cactgtctga gaatttccaa 2640

aactttaatg aactaactga cagcttcatg aaactgtcca ccaagatcaa gcagagaaaa 2700

taattaattt catgggacta aatgaactaa tgaggattgc tgattcttta aatgtcttgt 2760

ttcccagatt tcaggaaact ttttttcttt taagctatcc actcttacag caatttgata 2820

aaatatactt ttgtgaacaa aaattgagac atttacattt tctccctatg tggtcgctcc 2880

agacttggga aactattcat gaatatttat attgtatggt aatatagtta ttgcacaagt 2940

tcaataaaaa tctgctcttt gtataacaga aaaa 2974

<210> 12
<211> 17
<212> DNA
<213> Homo sapiens

<400> 12
   agatcgacaa cgcccgt 17

<210> 13
<211> 20
<212> DNA
<213> Homo sapiens

<400> 13
agagcctgtt ccgtctcaaa 20

<210> 14
<211> 19
<212> DNA
<213> Homo sapiens

<400> 14
ccagggagcg actgttgtc 19

<210> 15
<211> 24

<212>  DNA
<213>  Homo sapiens

<400>  15
ggaaatcaca ttctccaata aggg                                              24


<210>  16
<211>  24
<212>  DNA
<213>  Homo sapiens

<400>  16
agccagagcc tgcgtagcag tgct                                              24


<210>  17
<211>  25
<212>  DNA
<213>  Homo sapiens

<400>  17
ctgtcgtaca ctgtatgcat catca                                             25


<210>  18
<211>  20
<212>  DNA
<213>  Homo sapiens

<400>  18
gcagatgcct aattcccgaa                                                   20


<210>  19
<211>  19
<212>  DNA
<213>  Homo sapiens

<400>  19
caaaaggcgg ctgatcgat                                                    19


<210>  20
<211>  21
<212>  DNA
<213>  Homo sapiens

<400>  20
ggcgatcttc agctcatatg c                                                 21


<210>  21
<211>  22
<212>  DNA
<213>  Homo sapiens

<400>  21
cctccttggc catcttgtta ag                                                22


<210>  22
<211>  21
<212>  DNA
<213>  Homo sapiens

<400>  22
tcatttgctc aaagctggct g                                                21

<210>  23
<211>  16
<212>  DNA
<213>  Homo sapiens

<400>  23
gtgaaggcca cagcat                                                      16

<210>  24
<211>  19
<212>  DNA
<213>  Homo sapiens

<400>  24
ggaaatcaca ttctccaat                                                   19

<210>  25
<211>  17
<212>  DNA
<213>  Homo sapiens

<400>  25
gcagatgcct aattccc                                                     17

<210>  26
<211>  20
<212>  DNA
<213>  Homo sapiens

<400>  26
tgtcaacaac aaagattcca                                                  20

<210>  27
<211>  18
<212>  DNA
<213>  Homo sapiens

<400>  27
tctccgaaga gcttgttg                                                    18

<210>  28
<211>  17
<212>  DNA
<213>  Homo sapiens

<400>  28
agcccatcat tgttctg                                                     17

## Description

### CROSS REFERENCE TO RELATED APPLICATIONS

This application claims priority to United States Provisional Patent Application Nos. 60/586,599 and 60/587,019, both filed on July 9, 2004, each of which is incorporated herein by reference in its entirety.

### BACKGROUND

### 1. Field of the Invention

Provided are improved methods for diagnosing cancer cells in lymph nodes, along with compositions and apparatus useful in conducting those methods.

### 2. Description of the Related Art

Early detection of cancer typically leads to increased survival rates. Metastatic lesions commonly are detected by histological techniques, including immunohistochemical techniques. Metastasized cells typically infiltrate the lymph nodes, and, thus in most instances, certain sentinel lymph nodes (lymph nodes where metastasized cells typically first infiltrate), or staging lymph nodes (lymph nodes typically analyzed for presence of certain types of cancers), are recognized for each cancer type and are analyzed for the presence of lesions, including micrometastases. Trained histologists often can detect metastatic lesions visually after tissue from a sentinel or staging lymph node is sectioned and stained. Highly trained histologists often can visualize micrometasteses, but the ability to visualize such lesions varies from histologist-to-histologist.

In many surgical procedures to remove tumors, biopsies of sentinel lymph nodes are taken. The surgical procedure is then halted and the excised lymphatic tissue is then analyzed. Once it is determined that the tumor has metastasized, a second, more radical surgical procedure is performed, removing regional lymphatics. A rapid method for identifying tumors is therefore warranted, not only because more assays can be performed in a given time period, thereby increasing laboratory turnaround, but permitting accurate, intraoperative decisions to be made, rather than conducting a second surgical procedure. It is therefore desirable to identify useful diagnostics for malignancies, especially that permit rapid and/or intraoperative detection of lymphatic micrometastases.

### SUMMARY

The present invention relates to a diagnostic method for detecting the presence of cancer cells in a patient by identifying the expression of certain markers indicative of the presence of cancer cells.

In one embodiment, the present invention relates to a method of identifying the expression of markers indicative of the presence of breast cancer cells in a lymph node of a patient. The method comprises determining if an mRNA species specific to one or more of TACSTD1, CK19, MGB1, MGB2, PIP and CK7 is overabundant in an RNA sample prepared from the lymph node. The overabundance of the mRNA species is indicative of the presence of displaced breast cells in the lymph node.

In another embodiment, the present invention relates to a method for identifying the expression of markers indicative of the presence of lung cancer cells in a lymph node of a patient. The method comprises determining if an mRNA species specific to one or more of CEA, CK7, CK19, PVA, SCCA1.2 (SCCA1 +SCCA2), SFTPB and TACSTD1 is overabundant in an RNA sample prepared from the lymph node. The overabundance of the mRNA species is indicative of the presence of displaced lung cells in the lymph node.

In still another embodiment, the present invention relates to an article of manufacture comprising packaging material and one or more nucleic acids specific to one or more of CK7, CK19, MGB1, MGB2, PIP and TACSTD1. The packaging material comprises an indicia, for example and without limitation, a writing, illustration, label, tag, book, booklet and/or package insert, indicating that the one or more nucleic acids can be used in a method of identifying expression of markers indicative of the presence of breast cancer cells in a lymph node of a patient.

In a further embodiment, the present invention relates to an article of manufacture comprising packaging material and one or more nucleic acids specific to one or more of CEA, CK7, CK19, PVA, SCCA1.2, SFTPB and TACSTD1. The packaging material comprises an indicia indicating that the one or more nucleic acids can be used in a method of identifying expression of markers indicative of the presence of lung cancer cells in a lymph node of a patient.

In a still further embodiment, the present invention relates to a composition comprising one or more primers or probes specific to one or more of CK7, CK19, MGB1, MGB2, PIP and TACSTD1 and RNA extracted from the lymph node of a patient diagnosed with or suspected of having breast cancer, or a nucleic acid, or analog thereof, derived from the RNA.

In yet a further embodiment, the present invention relates to a composition comprising one or more primers or probes specific to one or more of CEA, CK7, CK19, PVA, SCCA1.2, SFTPB and TACSTD1 and RNA extracted from the lymph node of a patient diagnosed with or suspected of having lung cancer, or a nucleic acid, or analog thereof, derived from the RNA.

### BRIEF DESCRIPTION OF THE DRAWINGS

**Figure 1** is a listing of a cDNA sequence of the cytokeratin 7 (CK7) marker (SEQ ID NO: 1).
**Figure 2** is a listing of a cDNA sequence of the cytokeratin 19 (CK19) marker (SEQ ID NO: 2).
**Figure 3** is a listing of a cDNA sequence of the mammaglobin 1 (MGB1) marker (SEQ ID NO: 3).
**Figure 4** is a listing of a cDNA sequence of the mammaglobin 2 (MGB2) marker (SEQ ID NO: 4).
**Figure 5** is a listing of a cDNA sequence of the prolactin-inducible protein (PIP) marker (SEQ ID NO: 5).
**Figure 6** is a listing of a cDNA sequence of the pemphigus vulgaris (PVA) marker (SEQ ID NO: 6).
**Figure 7** is a listing of a cDNA sequence of the squamous cell carcinoma antigen 1 (SCCA1) marker (SEQ ID NO: 7).
**Figure 8** is a listing of a cDNA sequence of the squamous cell carcinoma antigen 2 (SCCA2) marker (SEQ ID NO: 8).
**Figure 9** is a listing of a cDNA sequence of the surfactant, pulmonary-associated protein b (SFTPB) marker (SEQ ID NO: 9).
**Figure 10** is a listing of a cDNA sequence of the tumor-associated calcium signal transducer 1 (TACSTD1) marker (SEQ ID NO: 10).
**Figure 11** is a listing of a cDNA sequence of the carcinoembryonic antigen-related cell adhesion molecule 5 (CEA) marker (SEQ ID NO: 11).
**Figure 12** is a scatter plot showing the expression levels of CK7, CK19, MGB1, MGB2, PIP and TACSTD1 in primary tumor, tumor-positive lymph nodes and benign lymph nodes of a breast cancer patient.
Figures 13A-O provide scatter plots illustrating the ability of two-marker systems to distinguish between benign and malignant cells in a lymph node of a breast cancer patient (negative - gray circle; positive - black circle).
**Figure 14** is a scatter plot showing the expression levels of CEA, CK7, CK19, LUNX, PVA, SCCA 1.2, SFTPB and TACSTD1 in primary tumor, tumor-positive lymph nodes and benign lymph nodes of a lung cancer patient.
Figure 15A-BB provide scatter plots illustrating the ability of two-marker systems to distinguish between benign and malignant cells in a lymph node of a lung cancer patient (negative - circle; positive - "+").
**Figure 16** is a plot of the best combination of three markers for detecting lung cancer in different histological types, plotting the Fold Change-Positive Lymph Nodes PLN vs. Highest Benign Nodes BN (PVA - asterix; SFTPB - circle; and TACSTD1- triangle).
**Figure 17A** provides data obtained from the secondary screening set of lymph nodes on individual gene expression observed in primary tumors, benign and positive nodes. The horizontal line indicates the most accurate cutoff value calculated by a receiver-operator characteristic curve analysis. Classification characteristics of the individual markers are reported in Table I, below.
Figures 17B-E provide secondary screening set data on gene expression for potential two-marker combinations using a linear discriminator decision rule. As with the individual markers, the black line indicates the decision rule generated from the secondary screening set data that produces the most accurate characterization. Classification characteristics of the marker combinations are reported in Table I.

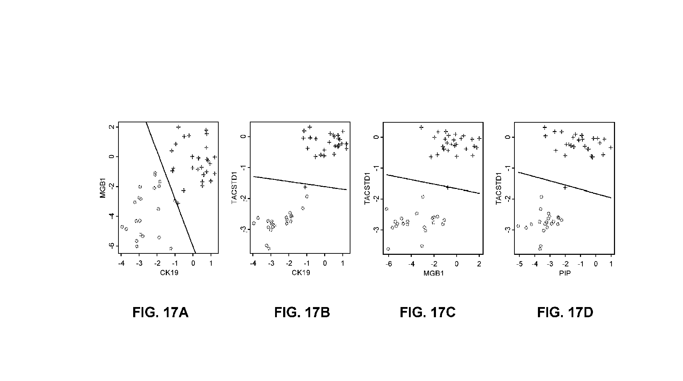

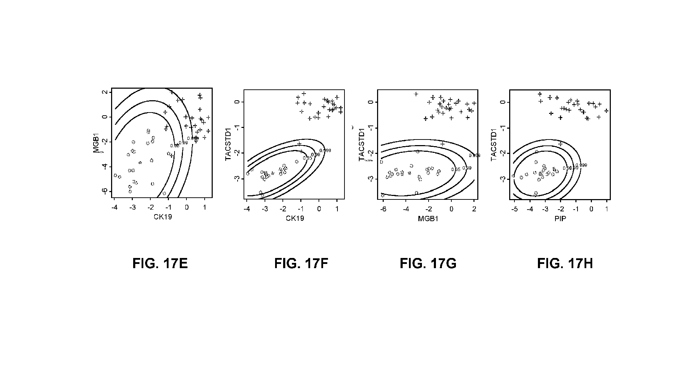

Figures 17F-I provide secondary screening set data on gene expression for potential two-marker combinations applying equal probability contour statistical analysis. Equal probability curves were generated around the mean expression value observed for the 2 markers in benign lymph nodes. This demonstrates that while the marker combination of CK19 and MGB1 accurately characterizes the lymph nodes (see Table I), the wide distribution of expression observed in benign nodes for these markers increases optimism in applying the decision rule. By this method of analysis, the marker combination of TACSTD1 and PIP more confidently characterizes the lymph nodes.
**Figure 18A** provides data obtained from the validation set of SLN on individual gene expression observed in negative and positive nodes. The horizontal line indicates the decision rule calculated from data obtained from the secondary screening set. Classification characteristics of the individual markers are reported in Table J.
Figures 18B-G provide validation set data on gene expression for two-marker combinations using linear discriminator decision rule for all potential marker pairs. As with the individual markers, the black line indicates the decision rule generated from the secondary screening set data that produces the most accurate characterization. Classification characteristics of the marker combinations are reported in Table J.
Figures 18H-M provide validation set data analyzed using the equal probability contours generated from the secondary screening set data. The relative levels of expression observed for all but one of the positive lymph nodes were well outside the 0.999 confidence contour. Some of the positive nodes are positive for only one marker (crosses located in the left upper or right lower quadrants), demonstrating that a 2 marker assay improves sensitivity while maintaining high specificity.
Figures 19A-B provides results of a fully automated, 2-marker QRT-PCR analysis of lymph nodes. By either linear decision rule analysis (Figure 19A) or equal probability contour analysis (Figure 19B), the assay accurately characterized all 18 lymph nodes (9 negative, 9 positive) evaluated.

### DETAILED DESCRIPTION

Provided are methods and compositions useful in identifying breast cancer and lung cancer cells, including micrometastases, in lymph nodes. Early detection of metastases typically is related to patient survival. Very small metastases often go undetected in histological study of lymph node biopsies, resulting in false negative results that result in decreased chances of patient survival. The nucleic acid detection assays described herein are much more discriminating than are histological studies in most instances (a few, excellent histologists are capable of identifying micrometastases in lymph node sections), and are robust and repeatable in the hands of any minimally-trained technician. Although the methods and compositions described herein are necessarily presented comprising expression of specific mRNA markers, this should be understood that it shall not be deemed to exclude methods and compositions comprising combinations of the specific markers and other markers known in the art.

To this end, a number of molecular markers are identified, that are expressed in certain cancer types, including breast cancer and lung cancer. These markers are markers specific to the tissue from which the particular cancer type arises and typically are not expressed, at least to the same levels, in lymphoid tissue. The presence and/or elevated expression of one or more of these markers in sentinel lymph node tissue is indicative of displaced cells in the lymphoid tissue, which correlates strongly with a cancer diagnosis.

As used herein, the terms "expression" and "expressed" mean production of a gene-specific mRNA by a cell. In the context of the present disclosure, a "marker" is a gene that is expressed abnormally in a lymphatic biopsy. In one embodiment, the markers described herein are mRNA species that are expressed in cells of a specific tumor source at a significantly higher level as compared to expression in lymphoid cells.

Expression levels of mRNA can be quantifyed by a number of methods. Traditional methods include Northern blot analysis. More recently, nucleic acid detection methods have been devised that facilitate quantification of transcripts. Examples of PCR methods are described in United States Patent Application No. 10/090,326 (US 10/090,326), incorporated herein by reference in its entirety. Other methods for determining expression levels of a given mRNA include isothermic amplification or detection assays and array technologies, as are known in the art, such as, without limitation, those described below.

The improved PCR methods described herein as well as in US 10/090,326, and other nucleic acid detection and amplification methods described herein and as are known in the art permit rapid detection of cancer cells in lymph node tissue. These rapid methods can be used intraoperatively, and also are useful in detecting rare nucleic acid species, even in multiplexed PCR reactions that concurrently detect a more prevalent control nucleic acid.

A typical PCR reaction includes multiple amplification steps, or cycles that selectively amplify a target nucleic acid species. Because detection of transcripts is necessary, the PCR reaction is coupled with a reverse transcription step (reverse transcription PCR, or RT-PCR). A typical PCR reaction includes three steps: a denaturing step in which a target nucleic acid is denatured; an annealing step in which a set of PCR primers (forward and backward primers) anneal to complementary DNA strands; and an elongation step in which a thermostable DNA polymerase elongates the primers. By repeating this step multiple times, a DNA fragment is amplified to produce an amplicon, corresponding to the target DNA sequence. Typical PCR reactions include 30 or more cycles of denaturation, annealing and elongation. In many cases, the annealing and elongation steps can be performed concurrently, that is at the same temperature, in which case the cycle contains only two steps.

The lengths of the denaturation, annealing and elongation stages may be any desirable length of time. However, in attempting to shorten the PCR amplification reaction to a time suitable for intraoperative diagnosis, the lengths of these steps can be in the seconds range, rather than the minutes range. The denaturation step may be conducted for times of one second or less. The annealing and elongation steps optimally are less than 10 seconds each, and when conducted at the same temperature, the combination annealing/elongation step may be less than 10 seconds. Use of recently developed amplification techniques, such as conducting the PCR reaction in a Rayleigh-Bénard convection cell, also can dramatically shorten the PCR reaction time beyond these time limits (see, Krishnan, My et al., "PCR in a Rayleigh-Bénard convection cell." Science 298:793 (2002), and Braun, D. et al., "Exponential DNA Replication by Lominar Convection," Physical Review Letters, 91:158103).

As described in US 10/090,326, each cycle may be shortened considerably without substantial deterioration of production of amplicons. Use of high concentrations of primers is helpful in shortening the PCR cycle time. High concentrations typically are greater than about 400nM, and often greater than about 800nM, though the optimal concentration of primers will vary somewhat from assay-to-assay. Sensitivity of RT-PCR assays may be enhanced by the use of a sensitive reverse transcriptase enzyme (described below) and/or high concentrations of reverse transcriptase primer to produce the initial target PCR template.

The specificity of any given PCR reaction relies heavily, but not exclusively, on the identity of the primer sets. The primer sets are pairs of forward and reverse oligonucleotide primers that anneal to a target DNA sequence to permit amplification of the target sequence, thereby producing a target sequence-specific amplicon. PCR primer sets can include two primers internal to the target sequence, or one primer internal to the target sequence and one specific to a target sequence that is ligated to the DNA or cDNA target, using a technique known as "ligation-anchored PCR" (Troutt, A.B., et al. (1992), "Ligation-anchored PCR: A Simple Amplification Technique with Single-sided Specificity," Proc. Natl. Acad. Sci. USA, 89:9823-9825).

As used herein, a "derivative" of a specified oligonucleotide is an oligonucleotide that binds to the same target sequence as the specified oligonucleotide and amplifies the same target sequence to produce essentially the same amplicon as the specified oligonucleotide but for differences between the specified oligonucleotide and its derivative. The derivative may differ from the specified oligonucleotide by insertion, deletion and/or substitution of any residue of the specified sequence so long as the derivative substantially retains the characteristics of the specified sequence in its use for the same purpose as the specified sequence.

As used herein, "reagents" for any assay or reaction, such as a reverse transcription and PCR, are any compound or composition that is added to the reaction mixture including, without limitation, enzyme(s), nucleotides or analogs thereof, primers and primer sets, probes, antibodies or other binding reagents, detectable labels or tags, buffers, salts and co-factors. As used herein, unless expressed otherwise, a "reaction mixture" for a given assay or reaction includes all necessary compounds and/or compositions necessary to perform that assay or reaction, even if those compounds or compositions are not expressly indicated. Reagents for many common assays or reactions, such as enzymatic reaction, are known in the art and typically are provided and/or suggested when the assay or reaction kit is sold.

As also described in US 10/090,326, multiplexed PCR assays may be optimized, or balanced, by time-shifting the production of amplicons, rather than by manipulating primer concentrations. This may be achieved by using two primer sets, each primer set having a different Tm so that a two-stage PCR assay can be performed, with different annealing and/or elongation temperatures for each stage to favor the production of one amplicon over another. This time and temperature shifting method permits optimal balancing of the multiplex reaction without the difficulties faced when manipulation of primer concentrations is used to balance the reaction. This technique is especially useful in a multiplex reaction where it is desirable to amplify a rare cDNA along with a control cDNA.

A quantitative reverse transcriptase polymerase chain reaction (QRT-PCR) for rapidly and accurately detecting low abundance RNA species in a population of RNA molecules (for example, and without limitation, total RNA or mRNA), includes the steps of: a) incubating an RNA sample with a reverse transcriptase and a high concentration of a target sequence-specific reverse transcriptase primer under conditions suitable to generate cDNA; b) subsequently adding suitable polymerase chain reaction (PCR) reagents to the reverse transcriptase reaction, including a high concentration of a PCR primer set specific to the cDNA and a thermostable DNA polymerase to the reverse transcriptase reaction, and c) cycling the PCR reaction for a desired number of cycles and under suitable conditions to generate PCR product ("amplicons") specific to the cDNA. By temporally separating the reverse transcriptase and the PCR reactions, and by using reverse transcriptase-optimized and PCR-optimized primers, excellent specificity is obtained. The reaction may be conducted in a single tube (all tubes, containers, vials, cells and the like in which a reaction is performed may be referred to herein, from time to time, generically, as a "reaction vessel"), removing a source of contamination typically found in two-tube reactions. These reaction conditions permit very rapid QRT-PCR reactions, typically on the order of 20 minutes from the beginning of the reverse transcriptase reaction to the end of a 40 cycle PCR reaction.

The reaction c) may be performed in the same tube as the reverse transcriptase reaction by adding sufficient reagents to the reverse transcriptase (RT) reaction to create good, or even optimal conditions for the PCR reaction to proceed. A single tube may be loaded, prior to the running of the reverse transcriptase reaction, with: 1) the reverse transcriptase reaction mixture, and 2) the PCR reaction mixture to be mixed with the cDNA mixture after the reverse transcriptase reaction is completed. The reverse transcriptase reaction mixture and the PCR reaction mixture may be physically separated by a solid, or semi-solid (including amorphous, glassy substances and waxy) barrier of a composition that melts at a temperature greater than the incubation temperature of the reverse transcriptase reaction, but below the denaturing temperature of the PCR reaction. The barrier composition may be hydrophobic in nature and forms a second phase with the RT and PCR reaction mixtures when in liquid form. One example of such a barrier composition is wax beads, commonly used in PCR reactions, such as the AMPLIWAX PCR GEM products commercially available from Applied Biosystems of Foster City, California.

Alternatively, the separation of the reverse transcriptase and the PCR reactions may be achieved by adding the PCR reagents, including the PCR primer set and thermostable DNA polymerase, after the reverse transcriptase reaction is completed. Preferably the PCR reagents, are added mechanically by a robotic or fluidic means to make sample contamination less likely and to remove human error.

The products of the QRT-PCR process may be compared after a fixed number of PCR cycles to determine the relative quantity of the RNA species as compared to a given reporter gene. One method of comparing the relative quantities of the products of the QRT-PCR process is by gel electrophoresis, for instance, by running the samples on a gel and detecting those samples by one of a number of known methods including, without limitation, Southern blotting and subsequent detection with a labeled probe, staining with ethidium bromide and incorporating fluorescent or radioactive tags in the amplicons.

However, the progress of the quantitative PCR reactions typically is monitored by determining the relative rates of amplicon production for each PCR primer set. Monitoring amplicon production may be achieved by a number of processes, including without limitation, fluorescent primers, fluorogenic probes and fluorescent dyes that bind double-stranded DNA. A common method is the fluorescent 5' nuclease assay. This method exploits the 5' nuclease activity of certain thermostable DNA polymerases (such as *Taq* or *Tfl* DNA polymerases) to cleave an oligomeric probe during the PCR process. The oligomer is selected to anneal to the amplified target sequence under elongation conditions. The probe typically has a fluorescent reporter on its 5' end and a fluorescent quencher of the reporter at the 3' end. So long as the oligomer is intact, the fluorescent signal from the reporter is quenched. However, when the oligomer is digested during the elongation process, the fluorescent reporter no longer is in proximity to the quencher. The relative accumulation of free fluorescent reporter for a given amplicon may be compared to the accumulation of the same amplicons for a control sample and/or to that of a control gene, such as β-actin or 18S rRNA to determine the relative abundance of a given cDNA product of a given RNA in a RNA population. Products and reagents for the fluorescent 5' nuclease assay are readily available commercially, for instance from Applied Biosystems.

Equipment and software also are readily available for monitoring amplicon accumulation in PCR and QRT-PCR according to the fluorescent 5' nuclease assay and other QPCR/QRT-PCR procedures, including the Smart Cycler, commercially available from Cepheid of Sunnyvale, California, the ABI Prism 7700 Sequence Detection System (TaqMan), commercially available from Applied Biosystems. A cartridge-based sample preparation system (GenXpert) combines a thermal cycler and fluorescent detection device having the capabilities of the Smart Cycler product with fluid circuits and processing elements capable of automatically extracting specific nucleic acids from a tissue sample and performing QPCR or QRT-PCR on the nucleic acid. The system uses disposable cartridges that can be configured and pre-loaded with a broad variety of reagents. Such a system can be configured to disrupt tissue and extract total RNA or mRNA from the sample. The reverse transcriptase reaction components can be added automatically to the RNA and the QPCR reaction components can be added automatically upon completion of the reverse transcriptase reaction.

Further, the PCR reaction may be monitored of production (or loss) of a particular fluorochrome from the reaction. When the fluorochrome levels reach (or fall to) a desired level, the automated system will automatically alter the PCR conditions. In one example, this is particularly useful in the multiplexed embodiment described above, where a more-abundant (control) target species is amplified by the first, lower Tm, primer set at a lower temperature than the less abundant species amplified by the second, higher Tm, primer set. In the first stage of the PCR amplification, the annealing temperature is lower than the effective Tm of the first primer set. The annealing temperature then is automatically raised above the effective Tm of the first primer set when production of the first amplicon by the first primer set is detected. In a system that automatically dispenses multiple reagents from a cartridge, such as the GeneXpert system, a first PCR reaction may be conducted at the first Tm and, when the first PCR reaction proceeds past a threshold level, a second primer with a different Tm is added, resulting in a sequential multiplexed reaction.

In the above-described reactions, the amounts of certain reverse transcriptase and the PCR reaction components typically are atypical in order to take advantage of the faster ramp times of some thermal cyclers. Specifically, the primer concentrations are very high. Typical gene-specific primer concentrations for reverse transcriptase reactions are less than about 20 nM. To achieve a rapid reverse transcriptase reaction on the order of one to two minutes, the reverse transcriptase primer concentration was raised to greater than 20 nM, preferably at least about 50 nM, and typically about 100 nM. Standard PCR primer concentrations range from 100 nM to 300 nM. Higher concentrations may be used in standard PCR reactions to compensate for Tm variations. However, the referenced primer concentrations are for circumstances where no Tm compensation is needed. Proportionately higher concentrations of primers may be empirically determined and used if Tm compensation is necessary or desired. To achieve rapid PCR reactions, the PCR primer concentrations typically are greater than 200 nM, preferably greater than about 500 nM and typically about 800 nM. Typically, the ratio of reverse transcriptase primer to PCR primer is about 1 to 8 or more. The increase in primer concentrations permitted PCR experiments of 40 cycles to be conducted in less than 20 minutes.

A sensitive reverse transcriptase may be preferred in certain circumstances where either low amounts of RNA are present or a target RNA is a low abundance RNA. By the term "sensitive reverse transcriptase," it is meant a reverse transcriptase capable of producing suitable PCR templates from low copy number transcripts for use as PCR templates. The sensitivity of the sensitive reverse transcriptase may derive from the physical nature of the enzyme, or from specific reaction conditions of the reverse transcriptase reaction mixture that produces the enhanced sensitivity. One example of a sensitive reverse transcriptase is SensiScript RT reverse transcriptase, commercially available from Qiagen, Inc. of Valencia, California. This reverse transcriptase is optimized for the production of cDNA from RNA samples of <50ng, but also has the ability to produce PCR templates from low copy number transcripts. In practice, in the assays described herein, adequate results were obtained for samples of up to, and even in excess of, about 400 ng RNA. Other sensitive reverse transcriptases having substantially similar ability to reverse transcribe low copy number transcripts would be equivalent sensitive reverse transcriptase for the purposes described herein. Notwithstanding the above, the ability of the sensitive reverse transcriptase to produce cDNA from low quantities of RNA is secondary to the ability of the enzyme, or enzyme reaction system to produce PCR templates from low copy number sequences.

As discussed above, the procedures described herein also may be used in multiplex QRT-PCR processes. In its broadest sense, a multiplex PCR process involves production of two or more amplicons in the same reaction vessel. Multiplex amplicons may be analyzed by gel electrophoresis and detection of the amplicons by one of a variety of methods, such as, without limitation ethidium bromide staining, Southern blotting and hybridization to probes, or by incorporating fluorescent or radioactive moieties into the amplicons and subsequently viewing the product on a gel. However, real-time monitoring of the production of two or more amplicons is preferred. The fluorescent 5' nuclease assay is the most common monitoring method. Equipment is now available (for example, the above-described Smart Cycler and TaqMan products) that permits the real-time monitoring of accumulation of two or more fluorescent reporters in the same tube. For multiplex monitoring of the fluorescent 5' nuclease assay, oligomers are provided corresponding to each amplicon species to be detected. The oligomer probe for each amplicon species has a fluorescent reporter with a different peak emission wavelength than the oligomer probe(s) for each other amplicons species. The accumulation of each unquenched fluorescent reporter can be monitored to determine the relative amounts of the target sequence corresponding to each amplicon.

In traditional multiplex QPCR and QRT-PCR procedures, the selection of PCR primer sets having similar annealing and elongation kinetics and similar sized amplicons are desirable. The design and selection of appropriate PCR primer sets is a process that is well known to a person skilled in the art. The process for identifying optimal PCR primer sets, and respective ratios thereof to achieve a balanced multiplex reaction also is known. By "balanced," it is meant that certain amplicon(s) do not out-compete the other amplicon(s) for resources, such as dNTPs or enzyme. For instance, by limiting the abundance of the PCR primers for the more abundant RNA species in an RT-PCR experiment will allow the detection of less abundant species. Equalization of the Tm (melting temperature) for all PCR primer sets also is encouraged. See, for instance, ABI PRISM 7700 Sequence Detection System User Bulletin #5, "Multiplex PCR with TaqMan VIC Probes", Applied Biosystems (1998/2001).

Despite the above, for very low copy number transcripts, it is difficult to design accurate multiplex PCR experiments, even by limiting the PCR primer sets for the more abundant control species. One solution to this problem is to run the PCR reaction for the low abundance RNA in a separate tube than the PCR reaction for the more abundant species. However, that strategy does not take advantage of the benefits of running a multiplex PCR experiment. A two-tube process has several drawbacks, including cost, the addition of more room for experimental error and the increased chance of sample contamination, which is critical in PCR assays.

A method has been described in WO 02/070751 for performing a multiplex PCR process, including QRT-PCR and QPCR, capable of detecting low copy number nucleic acid species along with one or more higher copy number species. The difference between low copy number and high copy number nucleic acid species is relative, but is referred to herein as a difference in the prevalence of a low (lower) copy number species and a high (higher) copy number species of at least about 30-fold, but more typically at least about 100-fold. For purposes herein, the relative prevalence of two nucleic acid species to be amplified is more salient than the relative prevalence of the two nucleic acid species in relation to other nucleic acid species in a given nucleic acid sample because other nucleic acid species in the nucleic acid sample do not directly compete with the species to be amplified for PCR resources.

As used herein, the prevalence of any given nucleic acid species in a given nucleic acid sample, prior to testing, is unknown. Thus, the "expected" number of copies of a given nucleic acid species in an nucleic acid sample often is used herein and is based on historical data on the prevalence of that species in nucleic acid samples. For any given pair of nucleic acid species, one would expect, based on previous determinations of the relative prevalence of the two species in a sample, the prevalence of each species to fall within a range. By determining these ranges one would determine the difference in the expected number of target sequences for each species. An mRNA species is identified as "overabundant" if it is present in statistically significant amounts over normal prevalence of the mRNA species in a sample from a normal patient or lymph node. As is abundantly illustrated in the examples and plots provided herein, a person of skill in the art would be able to ascertain statistically significant ranges or cutoffs for determining the precise definition of "overabundance" for any one or more mRNA species.

The multiplex method involves performing a two- (or more) stage PCR amplification, permitting modulation of the relative rate of production of a first amplicon by a first primer set and a second amplicon by a second primer set during the respective amplification stages. By this method, PCR amplifications to produce amplicons directed to a lower abundance nucleic acid species are effectively "balanced" with PCR amplifications to produce amplicons directed to a higher abundance nucleic acid species. Separating the reaction into two or more temporal stages may be achieved by omitting the PCR primer set for any amplicons that are not to be produced in the first amplification stage. This is best achieved through use of automated processes, such as the GenXpert prototype system described above. Two or more separate amplification stages may be used to tailor and balance multiplex assays, along with, or to the exclusion of tailoring the concentration of the respective primer sets.

A second method for temporally separating the PCR amplification process into two or more stages is to select PCR primer sets with variation in their respective Tm. In one example, primers for a lower copy number nucleic acid species would have a higher Tm (Tm₁) than primers for a higher abundance species (Tm₂). In this process, the first stage of PCR amplification is conducted for a predetermined number of cycles at a temperature sufficiently higher than Tm₂ so that there is substantially no amplification of the higher abundance species. After the first stage of amplification, the annealing and elongation steps of the PCR reaction are conducted at a lower temperature, typically about Tm₂, so that both the lower abundance and the higher abundance amplimers are amplified. It should be noted that Tm, as used herein and unless otherwise noted, refers to "effective Tm," which is the Tm for any given primer in a given reaction mix, which depends on factors, including, without limitation, the nucleic acid sequence of the primer and the primer concentration in the reaction mixture.

It should be noted that PCR amplification is a dynamic process. When using temperature to modulate the respective PCR reactions in a multiplex PCR reaction, the higher temperature annealing stage may be carried out at any temperature typically ranging from just above the lower Tm to just below the higher Tm, so long as the reaction favors production of the amplicon by the higher Tm primer set. Similarly, the annealing for the lower temperature reaction typically is at any temperature below the Tm of the low temperature primer set.

In the example provided above, in the higher temperature stage the amplicon for the low abundance RNA is amplified at a rate faster than that the amplicon for the higher abundance RNA (and preferably to the substantial exclusion of production of the second amplicon), so that, prior to the second amplification stage, where it is desirable that amplification of all amplicons proceeds in a substantially balanced manner, the amplicon for the lower abundance RNA is of sufficient abundance that the amplification of the higher abundance RNA does not interfere with the amplification of the amplicon for the lower abundance RNA. In the first stage of amplification, when the amplicon for the low abundance nucleic acid is preferentially amplified, the annealing and elongation steps may be performed above Tm₁ to gain specificity over efficiency (during the second stage of the amplification, since there is a relatively large number of low abundance nucleic acid amplicons, selectivity no longer is a significant issue, and efficiency of amplicon production is preferred). It, therefore, should be noted that although favorable in many instances, the temperature variations may not necessarily result in the complete shutdown of one amplification reaction over another.

In another variation of the above-described amplification reaction, a first primer set with a first Tm may target a more-abundant template sequence (for instance, the control template sequence) and a second primer set with a higher Tm may target a less-abundant template sequence. In this case, the more-abundant template and the less-abundant template may both be amplified in a first stage at a temperature below the (lower) Tm of the first primer set. When a threshold amount of amplicon corresponding to the more abundant template is reached, the annealing and/or elongation temperature of the reaction is raised above the Tm of the first primer set, but below the higher Tm of the second primer set to effectively shut down amplification of the more abundant template.

Selection of three or more sets of PCR primer sets having three or more different Tms (for instance, Tm₁ > Tm₂ > Tm₃) can be used to amplify sequences of varying abundance in a stepwise manner, so long as the differences in the Tms are sufficiently large to permit preferential amplification of desired sequences to the substantial exclusion of undesired sequences for a desired number of cycles. In that process, the lowest abundance sequences are amplified in a first stage for a predetermined number of cycles. Next, the lowest abundance and the lesser abundance sequences are amplified in a second stage for a predetermined number of cycles. Lastly, all sequences are amplified in a third stage. As with the two-stage reaction described above, the minimum temperature for each stage may vary, depending on the relative efficiencies of each single amplification reaction of the multiplex reaction. It should be recognized that two or more amplimers may have substantially the same Tm, to permit amplification of more than one species of similar abundance at any stage of the amplification process. As with the two-stage reaction, the three-stage reaction may also proceed stepwise from amplification of the most abundant nucleic acid species at the lowest annealing temperature to amplification of the least abundant species at the highest annealing temperature.

By this sequential amplification method, an additional tool is provided for the "balancing" of multiplex PCR reactions besides the matching of Tms and using limiting amounts of one or more PCR primer sets. The exploitation of PCR primer sets with different Tms as a method for sequentially amplifying different amplicons may be preferred in certain circumstances to the sequential addition of additional primer sets. However, the use of temperature-dependent sequencing of multiplex PCR reactions may be coupled with the sequential physical addition of primer sets to a single reaction mixture.

An internal positive control that confirms the operation of a particular amplification reaction for a negative result also may be used. The internal positive controls (IPC) are DNA oligonucleotides that have the same primer sequences as the target gene (CEA or tyrosinase) but have a different internal probe sequence. Selected sites in the IPC's optionally may be synthesized with uracil instead of thymine so that contamination with the highly concentrated mimic could be controlled using uracil DNA glycosylase, if required. The IPCs maybe added to any PCR reaction mastermix in amounts that are determined empirically to give Ct values typically greater than the Ct values of the endogenous target of the primer set. The PCR assays are then performed according to standard protocols, and even when there is no endogenous target for the primer set, the IPC would be amplified, thereby verifying that the failure to amplify the target endogenous DNA is not a failure of the PCR reagents in the mastermix. In this embodiment, the IPC probe fluoresces differently than the probe for the endogenous sequences. A variation of this for use in RT-PCR reactions is where the IPC is an RNA and the RNA includes an RT primer sequence. In this embodiment, the IPC verifies function of both the RT and PCR reactions. Both RNA and DNA IPCs (with different corresponding probes) may also be employed to differentiate difficulties in the RT and PCR reactions.

The rapid QRT-PCR protocols described herein may be run in about 20 minutes. This short time period permits the assay to be run intraoperatively so that a surgeon can decide on a surgical course during a single operation (typically the patient will remain anesthetized and/or otherwise sedated in a single "operation", though there may be a waiting period between when the sample to be tested is obtained and the time the interoperative assay is complete), rather than requiring a second operation, or requiring the surgeon to perform unneeded or overly broad prophylactic procedures. For instance, in the surgical evaluation of certain cancers, including breast cancer, melanoma, lung cancer, esophageal cancer and colon cancer, tumors and sentinel lymph nodes are removed in a first operation. The sentinel nodes are later evaluated for micrometastases, and, when micrometastases are detected in a patient's sentinel lymph node, the patient will need a second operation, thereby increasing the patient's surgical risks and patient discomfort associated with multiple operations. With the ability to determine the expression levels of certain tumor-specific markers described herein in less than 30 minutes with increased accuracy, a physician can make an immediate decision on how to proceed without requiring the patient to leave the operating room or associated facilities. The rapid test also is applicable to needle biopsies taken in a physician's office. A patient need not wait for days to get the results of a biopsy (such as a needle biopsy of a tumor or lymph node), but can now get more accurate results in a very short time.

As used herein, in the context of gene expression analysis, a probe is "specific to" a gene or transcript if under reaction conditions it can hybrizide specifically to transcripts of that gene within a sample, or sequences complementary thereto, and not to other transcripts. Thus, in a diagnostic assay, a probe is specific to a gene if it can bind to a specific transcript or desired family of transcripts in mRNA extracted from a specimen, to the practical exclusion (does not interfere substantially with the detection assay) of other transcripts. In a PCR assay, primers are specific to a gene if they specifically amplify a sequence of that gene, to the practical exclusion of other sequences in a sample.

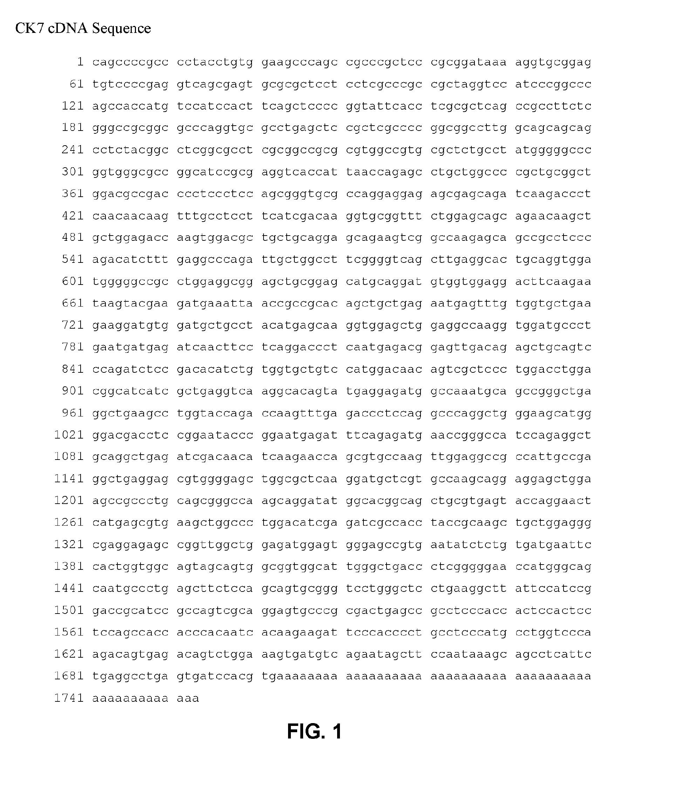

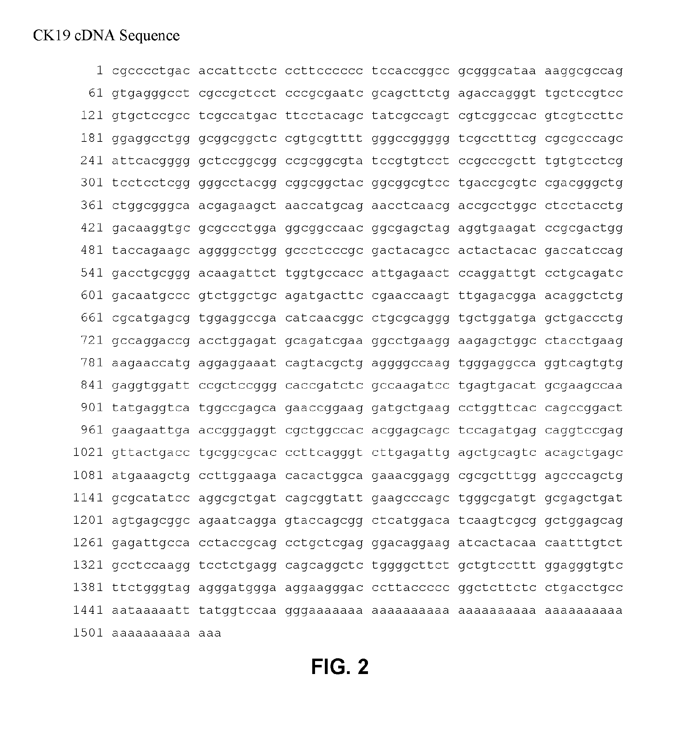

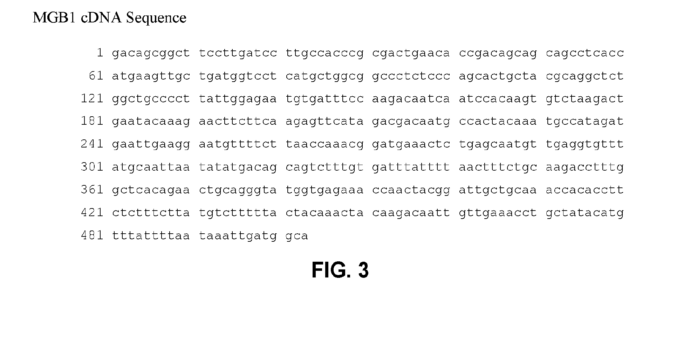

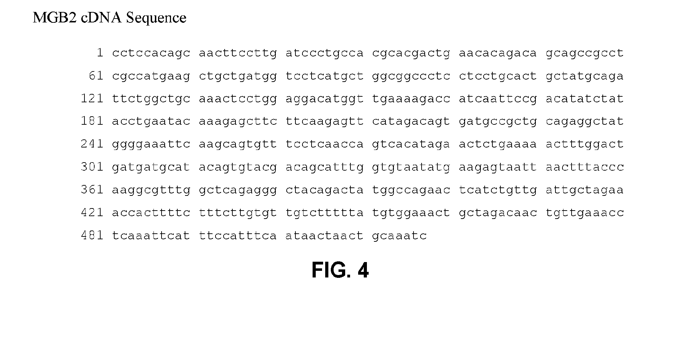

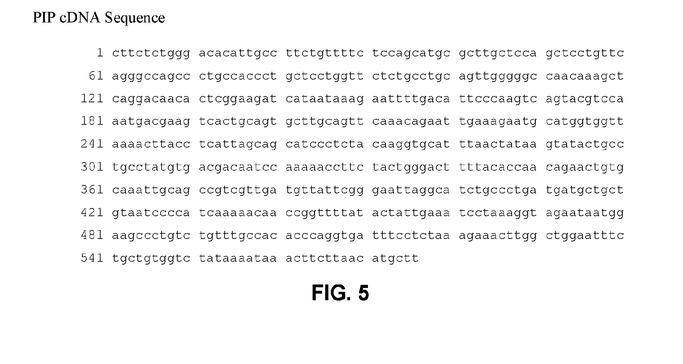

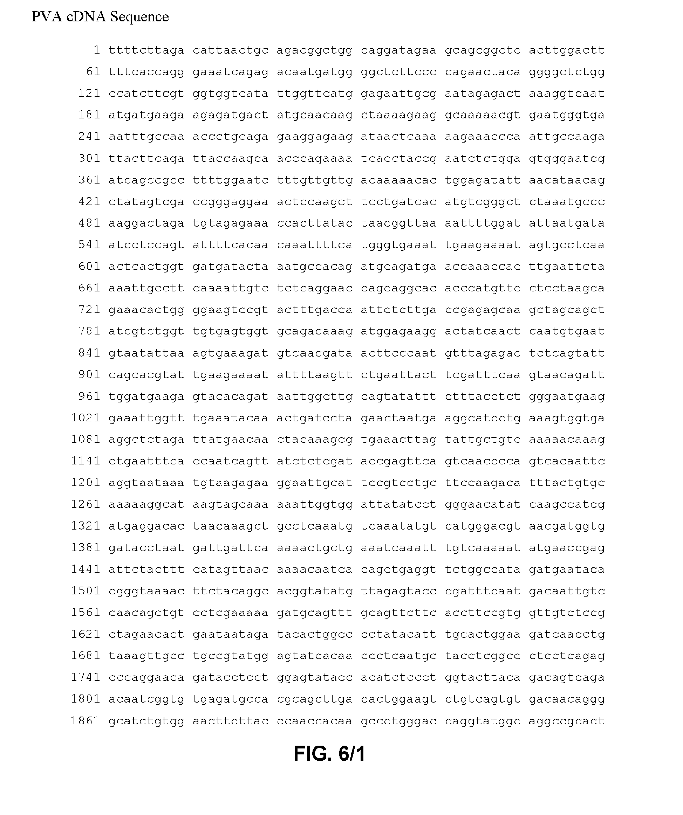

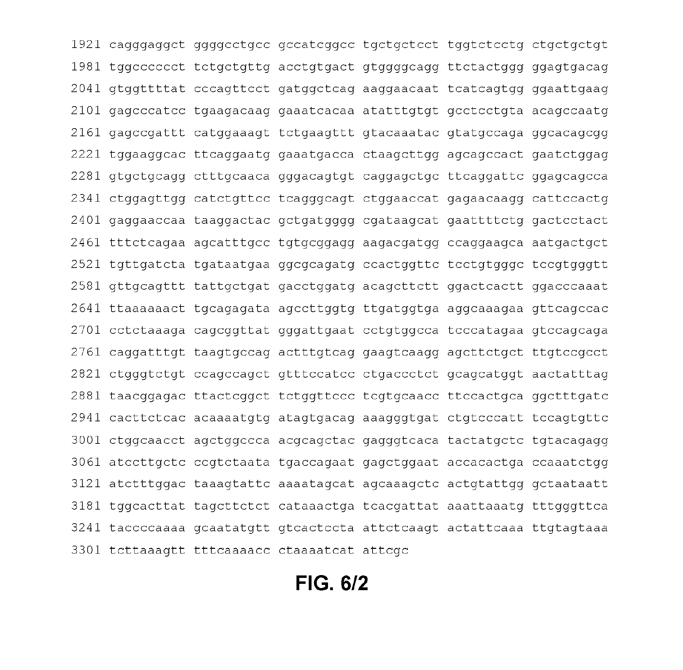

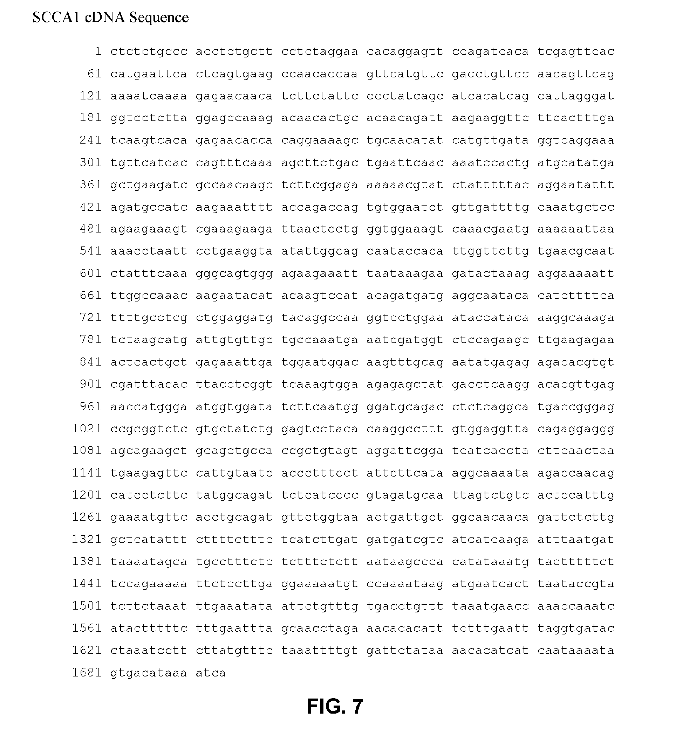

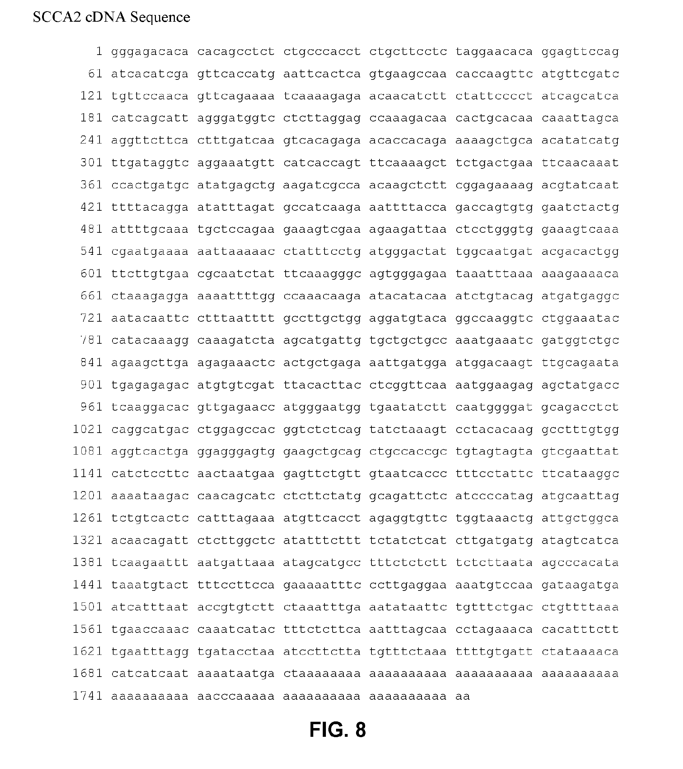

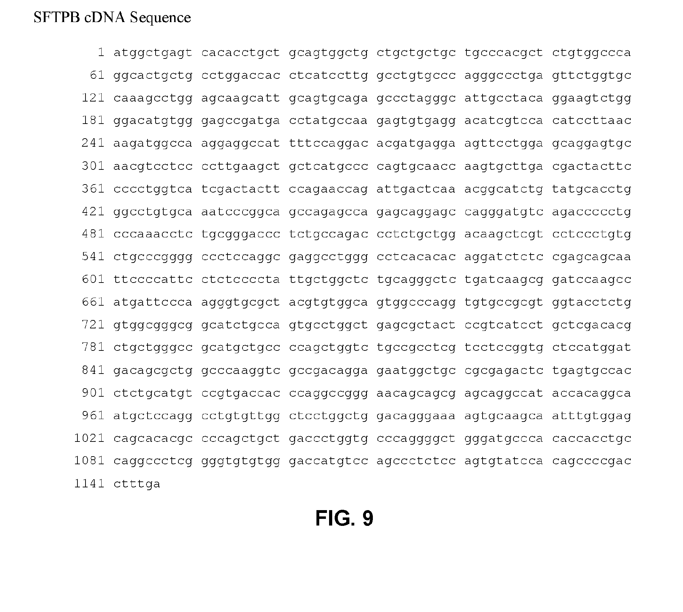

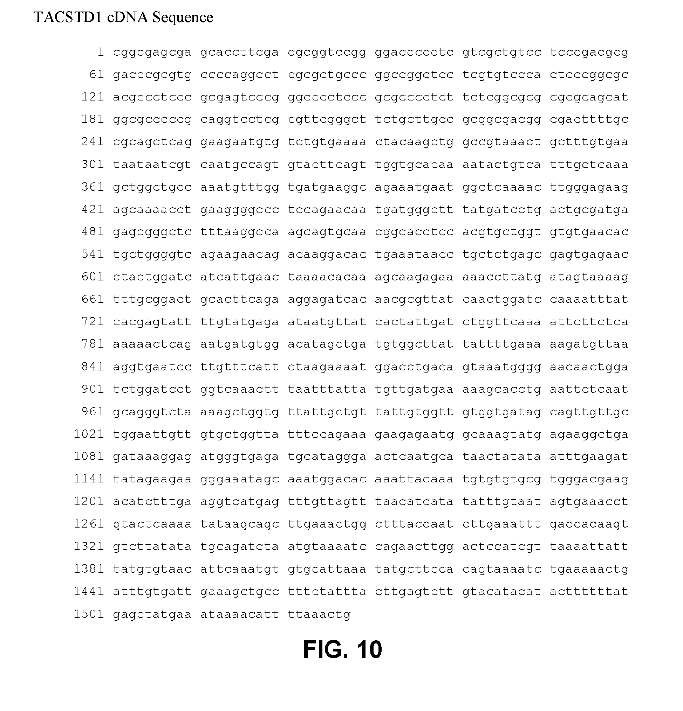

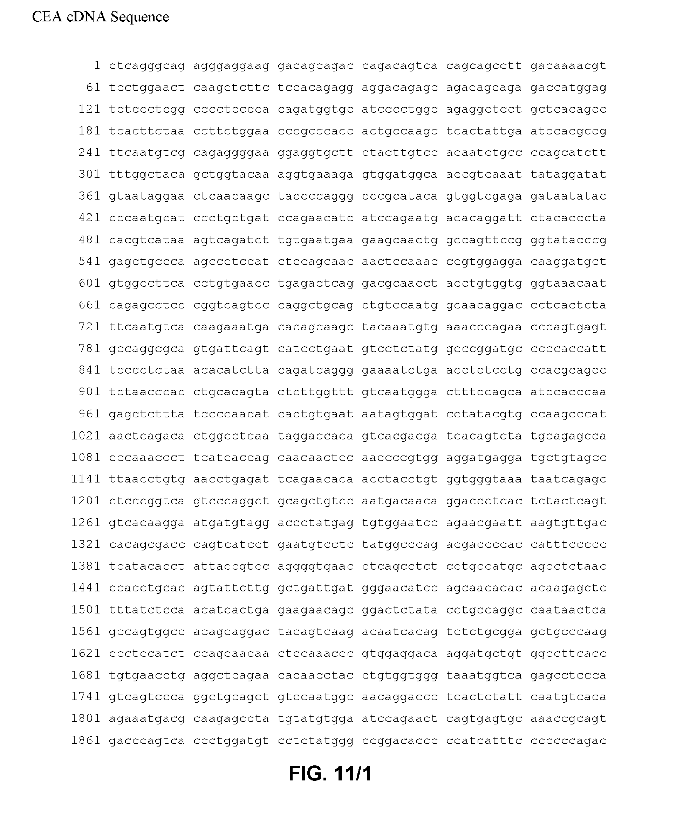

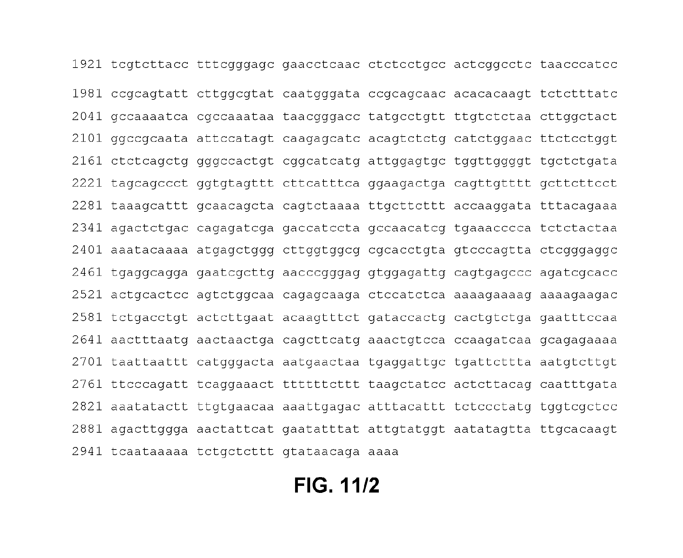

Table B provides primer and probe sequences for the mRNA quantification assays described and depicted in the Examples and Figures. Figures 1-11 provide non-limiting examples of cDNA sequences of the various mRNA species detected in the Examples. Although the sequences provided in Table B were found effective in the assays described in the examples, other primers and probes would likely be equally suited for use in the QRT-PCR and other mRNA detection and quantification assays, either described herein or as are known in the art. Design of alternate primer and probe sets for PCR assays, as well as for other mRNA detection assays is well within the abilities of one of average skill in the art. For example and without limitation, a number of computer software programs will generate primers and primer sets for PCR assays from cDNA sequences according to specified parameters. Non limiting examples of such software include, NetPrimer and Primer Premier 5, commercially available from PREMIER Biosoft International of Palo Alto, California, which also provides primer and probe design software for molecular beacon and array assays. Primers and/or probes for two or more different mRNAs can be identified, for example and without limitation, by aligning the two or more target sequences according to standard methods, determining common sequences between the two or more mRNAs and entering the common sequences into a suitable primer design computer program.

As used herein, a "primer or probe" for detecting a specific mRNA species is any primer, primer set and/or probe that can be utilized to detect and/or quantify the specific mRNA species. An "mRNA species" can be a single mRNA species, corresponding to a single mRNA expression product of a single gene, or can be multiple mRNAs that are detected by a single common primer and/or probe combination, such as the SCCA1.2 and MAGEA136-plex pecies described below.

In the commercialization of the methods described herein, certain kits for detection of specific nucleic acids will be particularly useful. A test typically comprises one or more reagents, such as, without limitation, nucleic acid primers or probes, packaged in a container, such as, without limitation, a vial, tube or bottle, in a package suitable for commercial distribution, such as, without limitation, a box, a sealed pouch, a blister pack and a carton. The package typically contains an indicia, for example and without limitation, a writing, illustration, label, book, booklet, tag and/or packaging insert, indicating that the packaged reagents can be used in a method for identifying expression or markers indicative of the presence of cancer cells in a lymph node of a patient. As used herein, "packaging materials" includes any article used in the packaging for distribution of reagents in a kit, including without limitation cointainers, vials, tubes, bottles, pouches, blister packaging, labels, tags, instruction sheets and package inserts. One example of such a kit would include reagents necessary for the one-tube QRT-PCR process described above. In one example, the kit would include the above-described reagents, including reverse transcriptase, a reverse transcriptase primer, a corresponding PCR primer set, a thermostable DNA polymerase, such as Taq polymerase, and a suitable fluorescent reporter, such as, without limitation, a probe for a fluorescent 5' nuclease assay, a molecular beacon probe, a single dye primer or a fluorescent dye specific to double-stranded DNA, such as ethidium bromide. The primers may be present in quantities that would yield the high concentrations described above. Thermostable DNA polymerases are commonly and commercially available from a variety of manufacturers. Additional materials in the kit may include: suitable reaction tubes or vials, a barrier composition, typically a wax bead, optionally including magnesium; reaction mixtures (typically 10X) for the reverse transcriptase and the PCR stages, including necessary buffers and reagents such as dNTPs; nuclease- or RNase- free water; RNase inhibitor; control nucleic acid(s) and/or any additional buffers, compounds, co-factors, ionic constituents, proteins and enzymes, polymers, and the like that may be used in reverse transcriptase and/or PCR stages of QRT-PCR reactions.

Components of a kit are packaged in any manner that is commercially practicable. For example, PCR primers and reverse transcriptase may be packaged individually to facilitate flexibility in configuring the assay, or together to increase ease of use and to reduce contamination. Similarly, buffers, salts and co-factors can be packaged separately or together.

The kits also may include reagents and mechanical components suitable for the manual or automated extraction of nucleic acid from a tissue sample. These reagents are known to those skilled in the art and typically are a matter of design choice. For instance, in one embodiment of an automated process, tissue is disrupted ultrasonically in a suitable lysis solution provided in the kit. The resultant lysate solution is then filtered and RNA is bound to RNA-binding magnetic beads also provided in the kit or cartridge. The bead-bound RNA is washed, and the RNA is eluted from the beads and placed into a suitable reverse transcriptase reaction mixture prior to the reverse transcriptase reaction. In automated processes, the choice of reagents and their mode of packaging (for instance in disposable single-use cartridges) typically are dictated by the physical configuration of the robotics and fluidics of the specific RNA extraction system, for example and without limitation, the GenXpert system. International Patent Publication Nos. WO 04/48931, WO 03/77055, WO 03/72253, WO 03/55973, WO 02/52030, WO 02/18902, WO 01/84463, WO 01/57253, WO 01/45845, WO 00/73413, WO 00/73412 and WO 00/72970 provide non-limiting examples of cartridge-based systems and related technology useful in the methods described herein.

The constituents of the kits may be packaged together or separately, and each constituent may be presented in one or more tubes or vials, or in cartridge form, as is appropriate. The constituents, independently or together, may be packaged in any useful state, including without limitation, in a dehydrated, lyophilized, a glassified or an aqueous state. The kits may take the physical form of a cartridge for use in automated processes, having two or more compartments including the above-described reagents. Suitable cartridges are disclosed for example in United States Patent Nos. 6,440,725, 6,431,476, 6,403,037 and 6,374,684.

Array technologies also can facilitate determining the expression level of two or more genes by facilitating performance of the desired reactions and their analysis by running multiple parallel reactions at the same time. One example of an array is the GeneChip® gene expression array, commercially available from Affymetrix, Inc. of Santa Clara, California. Patents illustrating array technology and uses therefor include, without limitation, United States Patent Nos. 6,040,138, 6,245,517, 6,251,601, 6,261,776, 6,306,643, 6,309,823, 6,346,413, 6,406,844 and 6,416,952. A plethora of other "array" patents exist, illustrating the multitude of physical forms a useful array can take. An "array", such as a "microarray" can be a substrate containing one or more binding reagents, typically in discrete physical locations, permitting high throughput analysis of the binding of a sample to the array. In the context of the methods described herein, an array contains probes specific to transcripts of one or more of the genes described herein affixed to a substrate. The probes can be nucleic acids or analogs thereof, as are known in the art. An array also can refer to a plurality of discrete reaction chambers, permitting multiple parallel reactions and detection events on a miniaturized scale.

As mentioned above, PCR-based technologies may be used to quantify mRNA levels in a given tissue sample. Other sequence-specific nucleic acid quantification methods may be more or less suited. In one embodiment, the nucleic acid quantification method is a rolling circle amplification method. Non-limiting examples of rolling circle amplification methods are described in U.S. Patents Nos. 5,854,003; 6,183,960; 6,344,329; and 6,210,884, each of which are incorporated herein by reference to the extent they teach methods for detecting and quantifying RNA species. In one embodiment, a padlock probe is employed to facilitate the rolling circle amplification process. (See Nilsson, M. et al. (2002), "Making Ends Meet in Genetic Analysis Using Padlock Probes," Human Mutation 19:410-415 and Schweitzer, B. et al (2001), "Combining Nucleic Acid Amplification and Detection," Current Opinion in Biotechnology, 12:21-27). A padlock probe is a linear oligonucleotide or polynucleotide designed to include one target-complementary sequence at each end, and which is designed such that the two ends are brought immediately next to each other upon hybridization to the target sequence. The probe also includes a spacer between the target-complementary sequences that includes a polymerase primer site and a site for binding to a probe, such as a molecular beacon probe, for detecting the padlock probe spacer sequence. If properly hybridized to an RNA template, the probe ends can then be joined by enzymatic DNA ligation to form a circular template that can be amplified by polymerase extension of a complementary primer. Thousands of concatemerized copies of the template can be generated by each primer, permitting detection and quantification of the original RNA template. Quantification can be automated by use, for example and without limitation, of a molecular beacon probe or other probe capable of detecting accumulation of a target sequence. By using padlock probes with different spacers to bind different molecular beacons that fluoresce a different color on binding to the amplified spacer, this automated reaction can be multiplexed. Padlock probe sequences target unique portions of the target RNA in order to ensure specific binding with limited or no cross-reactivity. RCA is an isothermic method in that the amplification is performed at one temperature.

Another isothermic method, for example and without limitation, is nucleic acid sequence-based amplification (NASBA). A typical NASBA reaction is initiated by the annealing of a first oligonucleotide primer to an RNA target in an RNA sample. The 3' end of the first primer is complementary to the target analyte; the 5' end encodes the T7 RNA polymerase promoter. After annealing, the primer is extended by reverse transcription (AMV-RT, for example) to produce a cDNA. The RNA is digested with RNase H, permitting a second primer (sense) to anneal to the cDNA strand, permitting the DNA polymerase activity of the reverse transcriptase to be engaged, producing a double-stranded cDNA copy of the original RNA template, with a functional T7 RNA polymerase promoter at one end. T7 polymerase is then used to produce an additional RNA template, which is further amplified, though in reverse order, according to the same procedure. A variety of other nucleic acid detection and/or amplification methods are known to those of skill in the art, including variations on the isothermic strand displacement, PCR and RCA methods described herein.

Further embodiments of the invention are disclosed in the clauses below.
1. A method of identifying expression of markers indicative of the presence of breast cancer cells in a lymph node of a patient, comprising determining if:
   (a) a first mRNA species specific to TACSTD1 is overabundant in an RNA sample prepared from the lymph node;
   (b) a first mRNA species specific to CK19 and a second mRNA species specific to one of PIP and MGB1 are overabundant in an RNA sample prepared from the lymph node; or
   (c) a first mRNA species specific to CK7 and a second mRNA species specific to one of PIP, MGB1 and MGB2 are overabundant in an RNA sample prepared from the lymph node, the overabundance of the mRNA species being indicative of the presence of displaced breast cells in the lymph node.
2. The method of clause 1, comprising determining if a first mRNA species specific to TACSTD1 is overabundant in an RNA sample prepared from the lymph node.
3. The method of clause 2, further comprising determining if a second mRNA species specific CK19 is overabundant in the RNA sample, the overabundance of the mRNA species being indicative of the presence of displaced breast cells in the lymph node.
4. The method of clause 2, further comprising determining if a second mRNA species specific to MGB1 is overabundant in the RNA sample, the overabundance of the mRNA species being indicative of the presence of displaced breast cells in the lymph node.
5. The method of clause 2, further comprising determining if a second mRNA species specific to MGB2 is overabundant in the RNA sample, the overabundance of the mRNA species being indicative of the presence of displaced breast cells in the lymph node.
6. The method of clause 2, further comprising determining if a second mRNA species specific to PIP is overabundant in the RNA sample, the overabundance of the mRNA species being indicative of the presence of displaced breast cells in the lymph node.
7. The method of clause 2, further comprising determining if a second mRNA species specific to CK7 is overabundant in the RNA sample, the overabundance of the mRNA species being indicative of the presence of displaced breast cells in the lymph node.
8. The method of clause 1, comprising determining if a first mRNA species specific to CK19 and a second mRNA species specific to one of PIP and MGB1 are overabundant in an RNA sample prepared from the lymph node, the overabundance of the mRNA species being indicative of the presence of displaced breast cells in the lymph node.
9. The method of clause 8, wherein the second mRNA species is specific to PIP.
10. The method of clause 8, wherein the second mRNA species is specific to MGB1.
11. The method of clause 1, comprising determining if a first mRNA species specific to CK7 and a second mRNA species specific to one of PIP, MGB1 and MGB2 are overabundant in an RNA sample prepared from the lymph node.
12. The method of clause 11, wherein the second mRNA species is specific to PIP.
13. The method of clause 11, wherein the second mRNA species is specific to MGB1.
14. The method of clause 11, wherein the second mRNA species is specific to MGB2.
15. The method of clause 1, comprising quantifying levels of the mRNA species in the RNA sample and determining if one or more of the mRNA species are overabundant in the RNA sample.
16. The method of clause 1, wherein a nucleic acid amplification assay is used to determine if the one or more mRNA species is overabundant in the RNA sample.
17. The method of clause 16, wherein the nucleic acid amplification assay is one of a PCR assay and an isothermic amplification assay.
18. The method of clause 17, wherein the nucleic acid amplification assay is an assay selected from the group consisting of RT-PCR, QRT-PCR, rolling circle amplification and nucleic acid sequences-based amplification assays.
19. The method of clause 17, wherein the assay is an RT-PCR assay.
20. The method of clause 19, wherein the RT-PCR assay uses one or more primer pairs specific to one or more of CK7, CK19, MGB1, MGB2, PIP and TACSTD1.
21. The method of clause 20, wherein the primer pairs consist essentially of at least about ten continguous nucleic acids of one or more of the CK7, CK19, MGB1, MGB2, PIP and TACSTD1 primers disclosed in Table B.
22. The method of clause 16, wherein the assay is a multiplex assay.
23. A method of identifying expression of markers indicative of the presence of lung cancer cells in a lymph node of a patient, comprising determining if a first mRNA species specific to one of CK7, CK19, PVA, SCCA1.2, SFTPB and TACSTD1 is overabundant in an RNA sample prepared from the lymph node, the overabundance of the mRNA species being indicative of the presence of displaced lung cells in the lymph node.
24. The method of clause 23, comprising determining if a first mRNA species specific to CK7 is overabundant in an RNA sample prepared from the lymph node, the overabundance of the mRNA species being indicative of the presence of displaced lung cells in the lymph node.
25. The method of clause 23, comprising determining if a first mRNA species specific to CK19 is overabundant in an RNA sample prepared from the lymph node, the overabundance of the mRNA species being indicative of the presence of displaced lung cells in the lymph node.
26. The method of clause 23, comprising determining if a first mRNA species specific to PVA is overabundant in an RNA sample prepared from the lymph node, the overabundance of the mRNA species being indicative of the presence of displaced lung cells in the lymph node.
27. The method of clause 23, comprising determining if a first mRNA species specific to SCCA1.2 is overabundant in an RNA sample prepared from the lymph node, the overabundance of the mRNA species being indicative of the presence of displaced lung cells in the lymph node.
28. The method of clause 23, comprising determining if a first mRNA species specific to SFTPB is overabundant in an RNA sample prepared from the lymph node, the overabundance of the mRNA species being indicative of the presence of displaced lung cells in the lymph node.
29. The method of clause 23, comprising determining if a first mRNA species specific to TACSTD1 is overabundant in an RNA sample prepared from the lymph node, the overabundance of the mRNA species being indicative of the presence of displaced lung cells in the lymph node.
30. The method of clause 29, further comprising determining if a second mRNA species specific to SFTPB is overabundant in the RNA sample, the overabundance of the mRNA species being indicative of the presence of lung cells in the lymph node.
31. The method of clause 30, further comprising determining if a third mRNA species specific to PVA is overabundant in the RNA sample, the overabundance of the mRNA species being indicative of the presence of lung cells in the lymph node.
32. The method of clause 30, further comprising determining if a third mRNA species specific to SCCA1.2 is overabundant in the RNA sample, the overabundance of the mRNA species being indicative of the presence of lung cells in the lymph node.
33. The method of clause 29, further comprising determining if a second mRNA species specific to PVA is overabundant in the RNA sample, the overabundance of the mRNA species being indicative of the presence of lung cells in the lymph node.
34. The method of clause 29, further comprising determining if an mRNA species specific to SCCA1.2 is overabundant in the RNA sample, the overabundance of the mRNA species being indicative of the presence of lung cells in the lymph node.
35. The method of clause 23, further comprising determining if one or more additional mRNA species, different from the first mRNA species, specific to one or more of CEA, CK7, CK19, PVA, SCCA1.2, SFTPB and TACSTD1 is overabundant in the RNA sample, the overabundance of the first mRNA species and the one or more additional mRNA species being indicative of the presence of lung cells in the lymph node.
36. The method of clause 23, comprising quantifying levels of the mRNA species in the RNA sample and determining if one or more of the mRNA species are overabundant in the RNA sample.
37. The method of clause 23, wherein a nucleic acid amplification assay is used to determine if the one or more mRNA species is overabundant in the RNA sample.
38. The method of clause 37, wherein the nucleic acid amplification assay is one of a PCR assay and an isothermic amplification assay.
39. The method of clause 38, wherein the nucleic acid amplification assay is an assay selected from the group consisting of RT-PCR, QRT-PCR, rolling circle amplification and nucleic acid sequences-based amplification assays.
40. The method of clause 38, wherein the assay is an RT-PCR assay.
41. The method of clause 40, wherein the RT-PCR assay uses one or more primer pairs specific to one or more of CEA, CK7, CK19, PVA, SCCA1.2, SFTPB and TACSTD1 .
42. The method of clause 41 , wherein the primer pairs consist essentially of at least about ten contiguous nucleic acids of one or more of CEA, CK7, CK19, PVA, SCCA1.2, SFTPB and TACSTD1 primers disclosed in Table B.
43. The method of clause 37, wherein the assay is a multiplex assay.
44. An article of manufacture comprising packaging material and:
   (a) one or more nucleic acids specific to one or more of CEA, CK19, MGB1, MGB2, PIP and TACSTD1, wherein the packaging material comprises an indicia indicating that the one or more nucleic acids can be used in a method of identifying expression of markers indicative of the presence of breast cancer cells in a lymph node of a patient; or
   (b) one or more nucleic acids specific to one or more of CEA, CK7, CK19, PVA, SCCA1.2, SFTPB and TACSTD1, wherein the packaging material comprises an indicia indicating that the one or more nucleic acids can be used in a method of identifying expression of markers indicative of the presence of lung cancer cells in a lymph node of a patient.
45. The article of manufacture of clause 44, comprising one or more nucleic acids specific to one or more of CEA, CK19, MGB1, MGB2, PIP and TACSTD1, wherein the packaging material comprises an indicia indicating that the one or more nucleic acids can be used in a method of identifying expression of markers indicative of the presence of breast cancer cells in a lymph node of a patient
46. The article of manufacture of clause 44, comprising one or more nucleic acids specific to one or more of CEA, CK7, CK19, PVA, SCCA1.2, SFTPB and TACSTD1, wherein the packaging material comprises an indicia indicating that the one or more nucleic acids can be used in a method of identifying expression of markers indicative of the presence of lung cancer cells in a lymph node of a patient.
47. The article of manufacture of clause 44, wherein the one or more nucleic acids are one or more primers for use in a sequence-specific nucleic acid detection or amplification assay.
48. The article of manufacture of clause 47, wherein the primers are one of PCR primer sets, NASBA primers and RCA primers.
49. The article of manufacture of clause 47, wherein the primers are PCR primer sets.
50. The article of manufacture of clause 44, wherein the one or more nucleic acids are attached to a substrate.
51. The article of manufacture of clause 50, wherein the substrate is an array of two or more of the one or more nucleic acids.
52. The article of manufacture of clause 44, wherein the one or more nucleic acids are probes.
53. The article of manufacture of clause 44, further comprising a detectable probe for use in detecting accumulation of a product of a sequence-specific nucleic acid detection or amplification assay utilizing the one or more primers.
54. The article of manufacture of clause 44, wherein the one or more nucleic acids are contained within a cartridge.
55. A composition comprising:
   (a) one or more primers or probes specific to one or more of CEA, CK19, MGB1, MGB2, PIP and TACSTD1 and RNA extracted from a lymph node of a patient diagnosed with or suspected of having breast cancer; or
   (b) one or more primers or probes specific to one or more of CEA, CK7, CK19, PVA, SCCA1.2, SFTPB and TACSTD1 and RNA extracted from a lymph node of a patient diagnosed with or suspected of having lung cancer.
56. The composition of clause 55, comprising one or more primers or probes specific to one or more of CEA, CK19, MGB1, MGB2, PIP and TACSTD1 and RNA extracted from a lymph node of a patient diagnosed with or suspected of having breast cancer.
57. The composition of clause 55, comprising one or more primers or probes specific to one or more of CEA, CK7, CK19, PVA, SCCA1.2, SFTPB and TACSTD1 and RNA extracted from a lymph node of a patient diagnosed with or suspected of having lung cancer.
58. The composition of clause 55, wherein the one or more primers or probes are attached to a substrate.
59. The composition of clause 58, wherein the substrate is an array of two or more of the one or more primers or probes.
60. The composition of clause 55, further comprising one or more of: a polymerase; a ribo- or deoxyribo-nucleotide, or an analog thereof; and a control nucleic acid.

### Example 1- General Materials and Methods

**Identification of Potential Markers.** An extensive literature and public database survey was conducted to identify any potential markers. Resources for this survey included PubMed, OMIM, UniGene (http://www.ncbi.nlm.nih.gov/), GeneCards (http://bioinfo.weizmann.ac.il/cards), and CGAP (http://cgap.nci.nih.gov). Survey criteria were somewhat flexible but the goal was to identify genes with moderate to high expression in tumors and low expression in normal lymph nodes. In addition, genes reported to be upregulated in tumors and genes with restricted tissue distribution were considered potentially useful. Finally, genes reported to be cancer-specific, such as the cancer testis antigens and hTERT, were evaluated.

Tissues and Pathological Evaluation. Tissue specimens were obtained from tissue banks at the University of Pittsburgh Medical Center through IRB approved protocols. All specimens were snap frozen in liquid nitrogen and later embedded in OCT for frozen sectioning. Twenty 5-micron sections were cut from each tissue for RNA isolation. In addition, sections were cut and placed on slides for H&E and IHC analysis at the beginning, middle (between the tenth and eleventh sections for RNA), and end of the sections for RNA isolation. All three H&E slides from each specimen underwent pathological review to confirm presence of tumor, percentage of tumor, and to identify the presence of any contaminating tissues. All of the unstained slides were stored at -20°C. Immunohistochemistry evaluation was performed using the AE1/AE3 antibody cocktail (DAKO, Carpinteria, CA), and Vector Elite ABC kit and Vector AEC Chromagen (Vecta Laboratories, Burlingame, CA). IHC was used as needed as needed to confirm the H&E histology.

**Screening Approach.** The screening was conducted in two phases. All potential markers entered the primary screening phase and expression was analyzed in 6 primary tumors and 10 benign lymph nodes obtained from patients without cancer (5 RNA pools with 2 lymph node RNA's per pool). Markers that showed good characteristics for lymph node metastasis detection passed into the secondary screening phase. The secondary screen consisted of expression analysis on 20-25 primary tumors, 20-25 histologically positive lymph nodes and 21 benign lymph nodes without cancer.

**RNA Isolation and cDNA Synthesis.** RNA was isolated using the RNeasy minikit (Qiagen, Valencia, CA) essentially as described by the manufacturer. The only modification was that we doubled the volume of lysis reagent and loaded the column in two steps. This was found to provide better RNA yield and purity, probably as a result of diluting out the OCT in the tissue sections. Reverse transcription was performed in 100-µl reaction volumes either with random hexamer priming or sequence-specific priming using a probe indicated in Table C, and Superscript II (Invitrogen, Carlsbad, CA) reverse transcriptase. For the primary screen, three reverse transcription reactions were performed, each with 500ng of RNA. The cDNA's were combined and QPCR was performed using the equivalent of 20ng RNA per reaction. For the secondary screen, the RNA input for primary tumors and positive nodes was also 500ng. For benign nodes however, the RNA input was 2000ng resulting in the equivalent of 80ng RNA per QPCR reaction.

**Quantitative PCR.** All quantitative PCR was performed on the ABI Prism 7700 Sequence Detection Instrument (Applied Biosystems, Foster City, CA). Relative expression of the marker genes was calculated using the delta-C_{T} methods previously described and with □-glucuronidase as the endogenous control gene. All assays were designed for use with 5' nuclease hybridization probes although the primary screening was performed using SYBER Green quantification in order to save cost. Assays were designed using the ABI Primer Express Version 2.0 software and where possible, amplicons spanned exon junctions in order to provide cDNA specificity. All primer pairs were tested for amplification specificity (generation of a single band on gels) at 60, 62 and 64°C annealing temperature. In addition, PCR efficiency was estimated using SYBER green quantification prior to use in the primary screen. Further optimization and more precise estimates of efficiency were performed with 5'nuclease probes for all assays used in the secondary screen.

A mixture of the Universal Human Reference RNA (Stratagene, La Jolla, CA) and RNAs from human placenta, thyroid, heart, colon, PCI13 cell line and SKBR3 cell line served as a universal positive expression control for all the genes in the marker screening process.

**Quantification with SYBER Green (Primary Screen).** For SYBR Green I-based QPCR, each 50µl reaction contained 1× TaqMan buffer A (Applied Biosystems), 300nM each dNTP, 3.5mM MgCl₂, 0.06 units/µl Amplitaq Gold (Applied Biosystems), 0.25X SYBR Green I (Molecular Probes, Eugene, OR) and 200nM each primer. The amplification program comprised 2-stages with an initial 95°C *Taq* activation stage for 12 min followed by 40 cycles of 95°C denaturation for 15 s, 60 or 62 or 64°C anneal/extend for 60 s and a 10 second data collection step at a temperature 2-4°C below the Tₘ of the specific PCR product being amplified (Tom B. Morrison, et al, 1998). After amplification, a melting curve analysis was performed by collecting fluorescence data while increasing the temperature from 60°C-95°C over 20 minutes.

**Quantification with 5' Nuclease Probes (Secondary Screen).** Probe-based QPCR was performed as described previously (Godfrey, et al., Clin Cancer Res. 2001 Dec., 7(12):4041-8). Briefly, reactions were performed with a probe concentration of 200nM and a 60 second anneal/extend phase at 60°C, or 62°C, or 64°C. The sequences of primers and probes (purchased from IDT, Coralville, IA) for genes evaluated in the secondary screen are listed in Table B, below.

**Data Analysis.** In the primary screen, data from the melt curve was analyzed using the ABI Prism 7700 Dissociation Curve Analysis 1.0 software (Applied Biosystems). The first derivative of the melting cure was used to determine the product Tₘ as well as to establish the presence of the specific product in each sample. In general, samples were analyzed in duplicate PCR reactions and the average Cₜ value was used in the expression analysis. However, in the secondary screen triplicate reactions were performed for each individual benign node and the lowest Cₜ value was used in the calculation of relative expression in order to obtain the highest value of background expression for the sample.

Cancer tissue-specific studies have been conducted, as described in the Examples below, in which a variety of molecular markers were identified as correlating with pathological states in cancers including breast cancer and lung cancer. Table A identifies genes used in the following studies. Table B provides PCR primer and TAQMAN probe sequences used in the quantitative PCR and RT-PCR amplifications described herein. Table C provides RT primer sequences as used instead of random hexamer primers. All PCR and RT-PCR reactions were conducted using standard methods. For all figures, T=primary tumor; PN=tumor-positive lymph nodes (by histological screening, that is, by review of H&E stained tissue and, when needed, by IHC, as described above); and BN=benign lymph nodes (by histological screening)

**Table A**

| **Marker** | **Accession No./ OMIM No.*** | **Official Gene Symbol** | **Official Gene Name** | **Alternative Gene Symbol** | **Alias** |
|---|---|---|---|---|---|
| CK7 | NM_005556/148059 | KRT7 | keratin 7 | K7, CK7, SCL, K2C7, MGC3625 | Sarcolectin; cytokeratin 7; type II mesothelial keratin K7; keratin, type II cytoskeletal 7; keratin, 55K type II cytoskeletal; keratin, simple epithelial type I, K7 |
| CK19 | NM_002276/148020 | KRT19 | keratin 19 | K19, CK19, KICS, MGC15366 | cytokeratin 19; keratin, type I, 40-kd; keratin, type I cytoskeletal 19; 40-kDa keratin intermediate filament precursor gene |
| MGB1 | NM_002411/605562 | SCGB2A2 | secretoglobin, family 2A, member 2 | MGB1, UGB2 | mammaglobin 1 |
| MGB2 | NM_002407/604398 | SCGB2A1 | secretoglobin, family 2A, member 1 | LPHC, MGB2, UGB3 | lipophilin C; mammaglobin 2; mammaglobin B |
| PIP | NM_002652/176720 | PIP | prolactin-induced protein | GP17, GCDFP-15 | prolactin-inducible protein |
| TACSTD1 | NM_002354/185535 | TACSTD1 | tumor-associated calcium signal transducer 1 | EGP, KSA, M4S1, MK-1, KS 1/4, EGP40, MIC18, TROP1, Ep-CAM, CO17-1A, GA733-2 | MK-1 antigen; antigen identified by monoclonal antibody AUA1; membrane component, chromosome 4, surface marker (35kD glycoprotein) |
| PVA | NM_001944/169615 | DSG3 | desmoglein 3 (pemphigus vulgaris antigen) | PVA, CDHF6 | pemphigus vulgaris antigen; 130-kD pemphigus vulgaris antigen |
| SCCA1 | NM_006919/600517 | SERPINB3 | serine (or cysteine) proteinase inhibitor, clade B (ovalbumin), member 3carcinoma antigen 1&2 | SCC, T4-A, SCCA1, SCCA-PD | squamous cell carcinoma antigen 1 |
| SCCA2 | NM_002974/600518 | SERPINB4 | serine (or cysteine) proteinase inhibitor, clade B (ovalbumin), member 4 | PI11, SCCA2, LEUPIN | leupin; squamous cell carcinoma antigen 2; protease inhibitor (leucine-serpin) |
| SFTPB | NM_000542/178640 | SFTPB | surfactant, pulmonary-associated protein b | SP-B, PSP-B, SFTB3,SFTP3 | Pulmonary surfactant-associated protein B, 18kD |

| | | | | | |
|---|---|---|---|---|---|
| *Online Mendelian Inheritance in Man (www.ncbi.nlm.nih.gov). | | | | | |

**Table B - Oligonucleotide primer and probe sequences used in secondary marker screening for all cancer types**

| **Gene** | **Oligonucleotide** | **Sequence (5' → 3')** | **Sequence Reference** |
|---|---|---|---|
| CK19 | Forward primer | AGATCGACAACGCCCGT | SEQ ID NO: 12 |
| | Reverse primer | AGAGCCTGTTCCGTCTCAAA | SEQ ID NO: 13 |
| | Probe | TGGCTGCAGATGACTTCCGAACCA | SEQ ID NO: 2, bases 614 to 637 |
| CK7 | Forward primer | CCCTCAATGAGACGGAGTTGA | SEQ ID NO: 1, bases 807 to 827 |
| | Reverse primer | CCAGGGAGCGACTGTTGTC | SEQ ID NO: 14 |
| | Probe | AGCTGCAGTCCCAGATCTCCGACACATC | SEQ ID NO: 1, bases 831 to 858 |
| MGB1 | Forward primer | GTTGCTGATGGTCCTCATGCT | SEQ ID NO: 3, bases 66 to 86 |
| | Reverse primer | GGAAATCACATTCTCCAATAAGGG | SEQ ID NO: 15 |
| | Probe | AGCCAGAGCCTGCGTAGCAGTGCT | SEQ ID NO: 16 |
| MGB2 | Forward primer | ATGCCGCTGCAGAGGCTAT | SEQ ID NO: 4, bases 222 to 240 |
| | Reverse primer | CTGTCGTACACTGTATGCATCATCA | SEQ ID NO: 17 |
| | Probe | TCAAGCAGTGTTTCCTCAACCAGTCACA | SEQ ID NO: 4, bases 249 to 276 |
| PIP | Forward primer | CTGGGACTTTTACACCAACAGAACT | SEQ ID NO: 5, bases 333 to 357 |
| | Reverse primer | GCAGATGCCTAATTCCCGAA | SEQ ID NO: 18 |
| | Probe | TGCAAATTGCAGCCGTCGTTGATGT | SEQ ID NO: 5, bases 359 to 383 |
| PVA | Forward primer | AAAGAAACCCAATTGCCAAGATTAC | SEQ ID NO: 6, bases 280 to 304 |
| | Reverse primer | CAAAAGGCGGCTGATCGAT | SEQ ID NO: 19 |
| | Probe | CCAAGCAACCCAGAAAATCACCTACCG | SEQ ID NO: 6, bases 314 to340 |
| SCCA1.2 | Forward primer | AAGCTGCAACATATCATGTTGATAGG | SEQ ID NO: 7, bases 267 to 292 |
| | Reverse primer | GGCGATCTTCAGCTCATATGC | SEQ ID NO: 20 |
| | Probe | TGTTCATCACCAGTTTCAAAAGCTTCTGACT | SEQ ID NO: 7, bases 301 to 331 |
| SFTPB | Forward primer | ACATGTGGGAGCCGATGAC | SEQ ID NO: 9, bases 183 to 201 |
| | Reverse primer | CCTCCTTGGCCATCTTGTTAAG | SEQ ID NO: 21 |
| | Probe | TGCCAAGAGTGTGAGGACATCGTCCAC | SEQ ID NO: 9, bases 205 to 231 |
| TACSTD1 | Forward primer | TCATTTGCTCAAAGCTGGCTG | SEQ ID NO: 10, bases 348 to 368 |
| | Reverse primer | GGTTTTGCTCTTCTCCCAAGTTT | SEQ ID NO: 22 |
| | Probe | AAATGTTTGGTGATGAAGGCAGAAATGAATGG | SEQ ID NO: 10, bases 371 to 402 |

| | | | |
|---|---|---|---|
| ^{A} A universal primer set designed to recognize transcripts of both SCCA1 AND SCCA2. | | | |

**Table C**

| **Gene Marker** | **RT Specific Primer (5' -> 3')** | **Sequence Reference** |
|---|---|---|
| CEA | GTGAAGGCCACAGCAT | SEQ ID NO: 23 |
| MGB1 | GGAAATCACATTCTCCAAT | SEQ ID NO: 24 |
| PIP | GCAGATGCCTAATTCCC | SEQ ID NO: 25 |
| PVA | TGTCAACAACAAAGATTCCA | SEQ ID NO: 26 |
| SCCA1.2 | TCTCCGAAGAGCTTGTTG | SEQ ID NO: 27 |
| TACSTD1 | AGCCCATCATTGTTCTG | SEQ ID NO: 28 |

### Example 2 - Breast Cancer

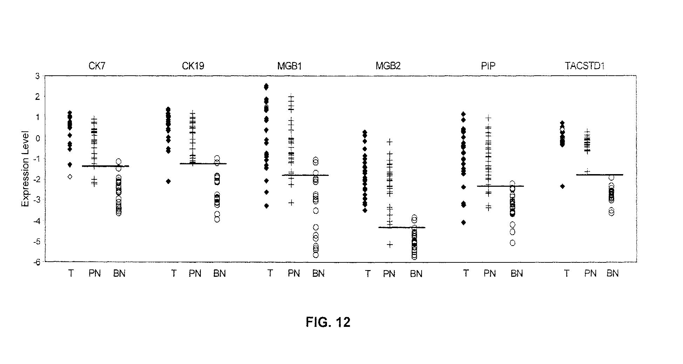

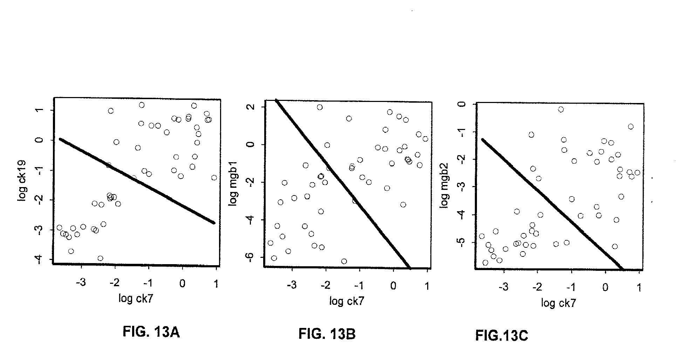

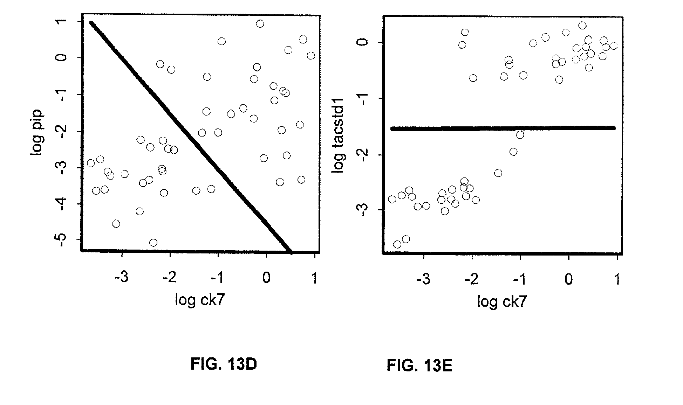

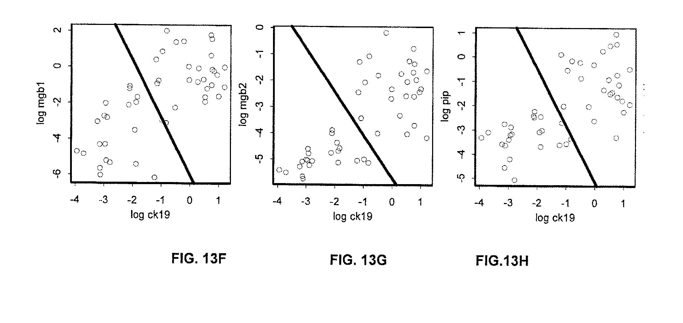

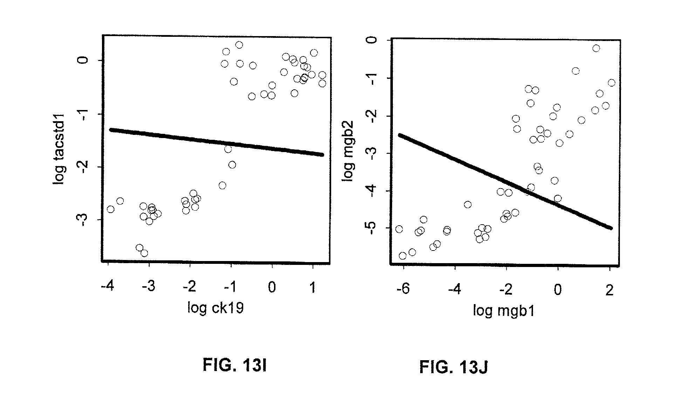

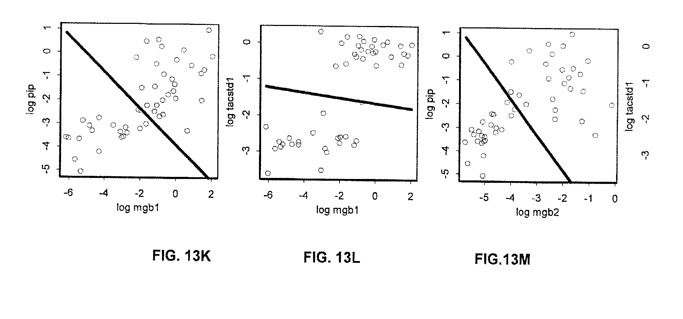

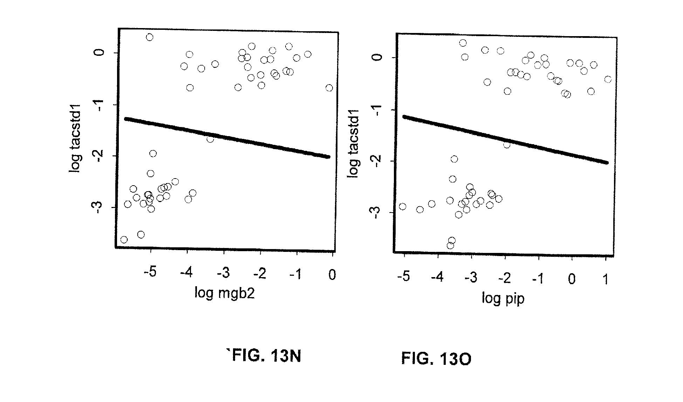

Expression levels of CK7, CK19, MGB1, MGB2, PIP, and TACSTD1 were determined by the methods described in Example 1. Figure 12 is a scatter plot showing the expression levels of CK7, CK19, MGB1, MGB2, PIP, and TACSTD1 in primary tumor, tumor-positive lymph nodes and benign lymph nodes. Figures 13A-O provide scatter plots illustrating the ability of two-marker systems to distinguish between benign and malignant cells in a lymph node. Tables D and E provide the raw data from which the graphs of Figures 12 and 13A-O were generated. This data illustrates the strong correlation of expression of CK7, CK19, MGB1, MGB2, PIP and TACSTD1 markers, alone or in combination, in sentinel lymph nodes with the presence of malignant cells arising from a breast cancer in the sentinel lymph nodes.

**Table D. Single Marker Prediction Characteristics for Breast Cancer**

| **Marker** | **Observed Data** | | | **Parametric Bootstrap Estimates*** | | | |
|---|---|---|---|---|---|---|---|
| | Sensitivity | Specificity | Classification Accuracy | Sensitivity | Specificity | Classification Accuracy | Classification Bias** |
| CK7 | .889 | .952 | .917 | .828 | .909 | .863 | .054 |
| CK19 | 1.0 | .952 | .979 | .997 | .891 | .951 | .028 |
| MGB1 | .926 | .857 | .896 | .903 | .748 | .836 | .060 |
| MGB2 | .963 | .905 | .938 | .943 | .834 | .895 | .043 |
| PIP | .852 | .952 | .896 | .814 | .892 | .848 | .048 |
| TACSTD1 | 1.0 | 1.0 | 1.0 | .999 | .956 | .980 | .020 |

| | | | | | | | |
|---|---|---|---|---|---|---|---|
| * 500 parametric bootstrap samples of 48 lymph node expression levels (27 positive, 21 benign were generated from the log-normal distribution and a new decision rule based on the most accurate cutoff was formulated each time (total of 500 bootstrap decision rules). The differences between classifying the original data and classifying the bootstrap data were averaged to form the estimate of bias in the re-substitution decision rule. The respective estimated bias was then subtracted from the sensitivity, specificity and classification of the original data to arrive at the bootstrap estimates. The bias in the estimated classification accuracy is shown in the last column ** Classification Bias = average difference in classification accuracies of the bootstrap decision rule applied to original data and the bootstrap decision rule applied to the bootstrap data. This estimates the optimism in using the original data to characterize the decision rule. | | | | | | | |

**Table E. Two Marker Prediction Characteristics for Breast Cancer**

| | Observed Data | | | Parametric Bootstrap Estimates* | | | Classification Bias** |
|---|---|---|---|---|---|---|---|
| | Sensitivity | Specificity | Classification Accuracy | Sensitivity | Specificity | Classification Accuracy | |
| CK7 + CK19 | 1.0 | .905 | .958 | .993 | .868 | .938 | .020 |
| CK7 + MGB 1 | .963 | 1.0 | .979 | .954 | 1.0 | .977 | .002 |
| CK7 + MGB2 | 1.0 | 1.0 | 1.0 | 1.0 | 1.0 | 1.0 | .000 |
| CK7 + PIP | .963 | 1.0 | .979 | .963 | 1.0 | .979 | .000 |
| CK7 + TACSTD1 | .963 | 1.0 | .979 | .928 | 1.0 | .959 | .020 |
| CK19 + MGB1 | 1.0 | 1.0 | 1.0 | .996 | 1.0 | 1.0 | .000 |
| CK19 + MGB2 | .963 | 1.0 | .979 | .945 | 1.0 | .975 | .004 |
| CK19 + PIP | .926 | 1.0 | .958 | .900 | 1.0 | .951 | .007 |
| CK19 + TACSTD1 | .963 | 1.0 | .979 | .928 | 1.0 | .960 | .019 |
| MGB1 + MGB2 | .889 | .952 | .917 | .853 | .925 | .885 | .032 |
| MGB1 + PIP | .963 | .905 | .938 | .963 | .876 | .934 | .004 |
| MGB1 + TACSTD1 | .963 | 1.0 | .979 | .942 | 1.0 | .967 | .012 |
| MGB2 + PIP | .926 | 1.0 | .958 | .915 | 1.0 | .953 | .005 |
| MGB2 + TACSTD1 | .963 | 1.0 | .979 | .930 | 1.0 | .961 | .018 |
| PIP + TACSTD1 | .963 | 1.0 | .979 | .929 | 1.0 | .960 | .017 |

| | | | | | | | |
|---|---|---|---|---|---|---|---|
| * 500 parametric bootstrap samples of 48 lymph node expression levels (27 positive, 21 benign were generated from the bivanate log-normal distribution and a new decision rule and a new decision rule formulated each time The differences between classifying the original data and classifying the bootstrap data were averaged to form the estimate of bias in the re-substitution decision rule The respective estimated bias was then subtracted from the sensitivity, specificity and classification accuracy of the original data to arrive at the bootstrap estimates The bias in the estimated classification accuracy is shown in the last column ** Classification Bias = average difference in classification accuracies of the bootstrap decision rule applied to original data and the bootstrap decision rule applied to the bootstrap data This estimates the optimism in using the original data to characterize the decision rule | | | | | | | |

### Example 3 - Lung Cancer

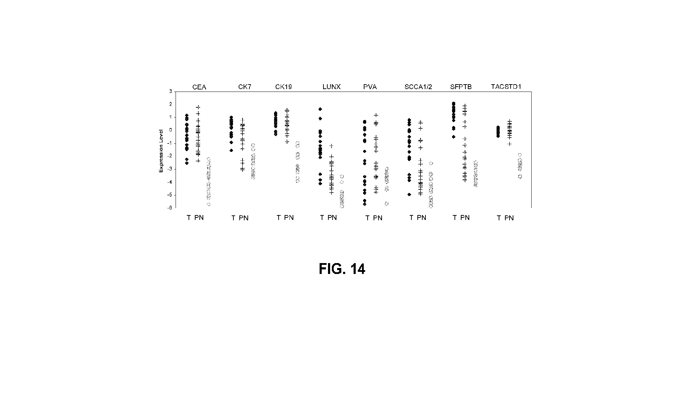

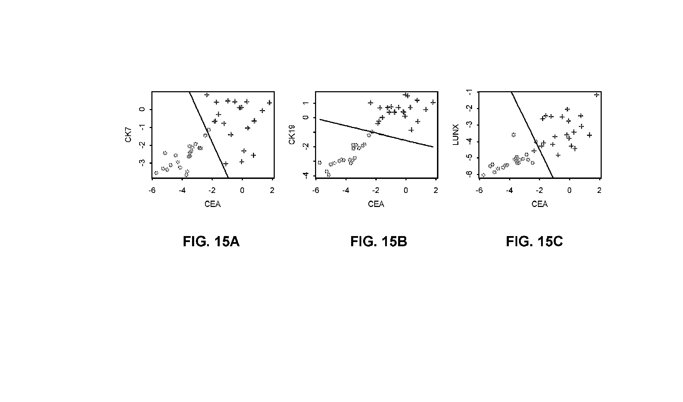

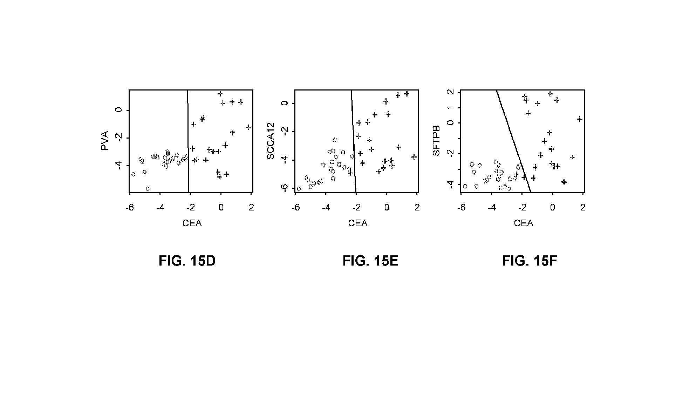

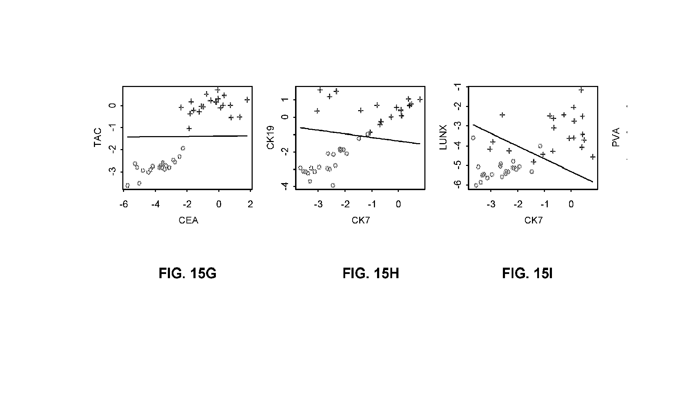

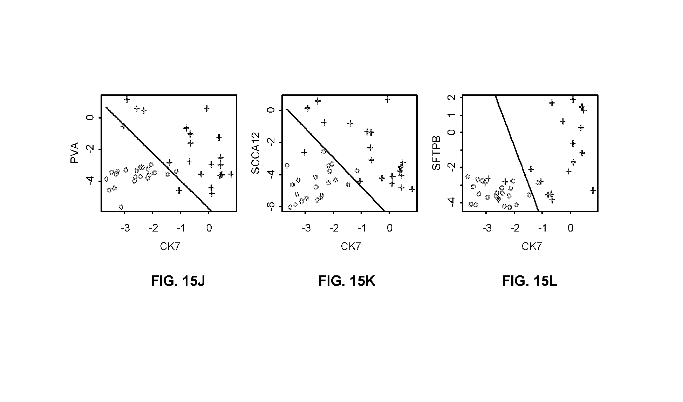

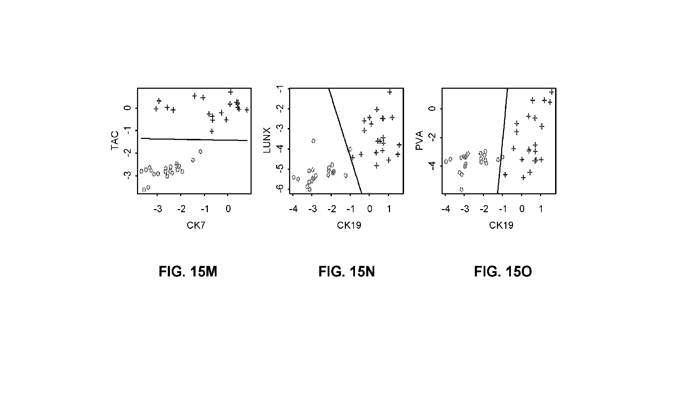

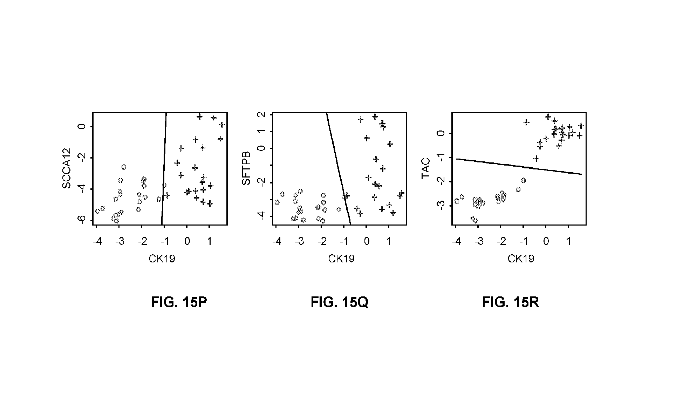

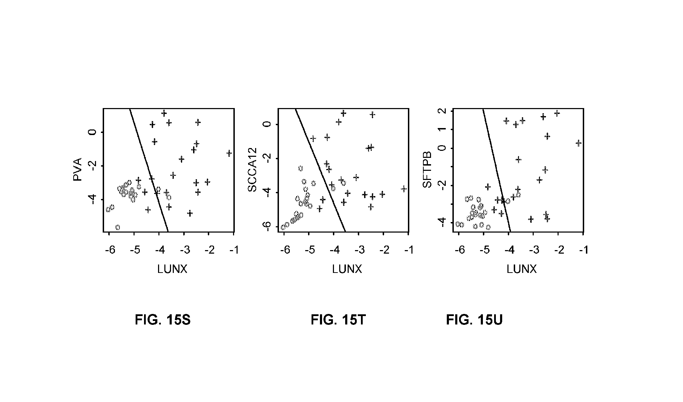

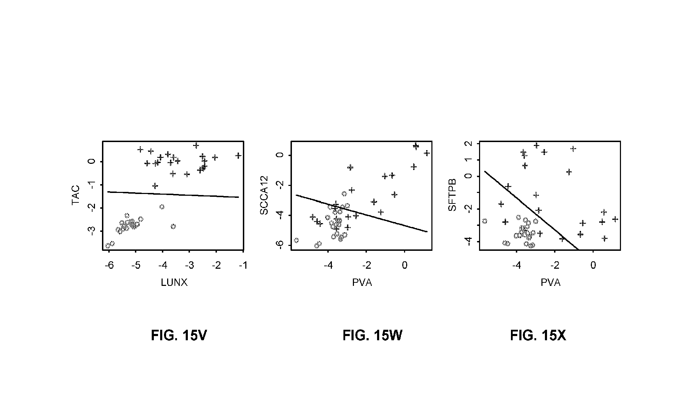

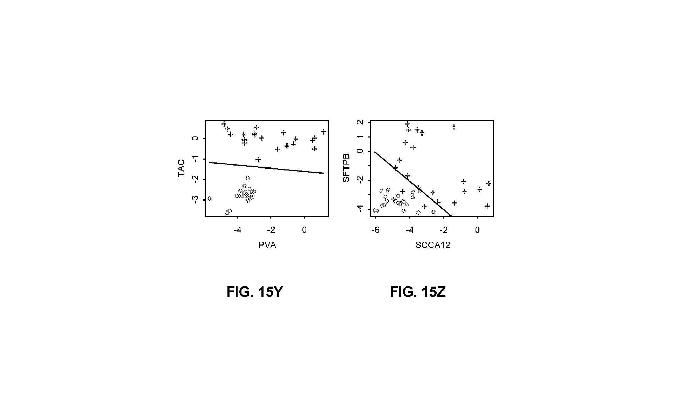

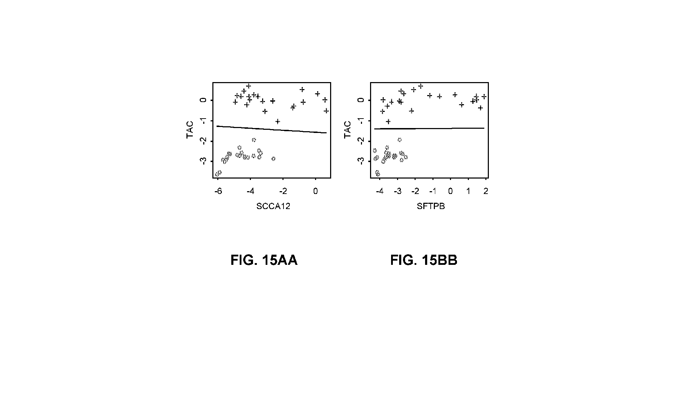

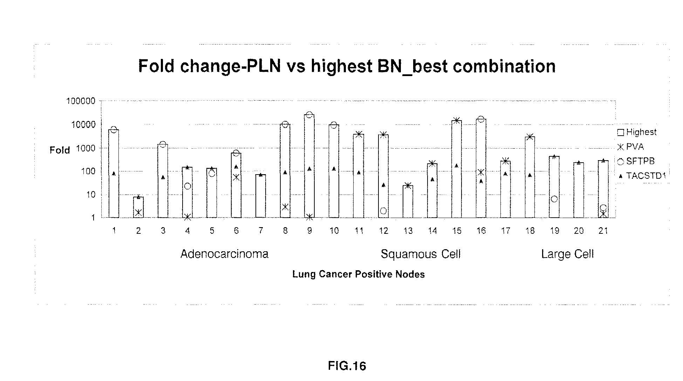

Expression levels of CEA, CK7, CK19, LUNX, PVA, SCCA1.2, SFTPB, and TACSTD1 were determined by the methods described in Example 1. Figure 14 is a scatter plot showing the expression levels of CEA, CK7, CK19, LUNX, PVA, SCCA1.2, SFTPB, and TACSTD1 in primary tumor, tumor-positive lymph nodes and benign lymph nodes. Figures 15A-BB provide scatter plots illustrating the ability of two-marker systems to distinguish between benign and malignant cells in a lymph node. Figure 16 is a plot of the best combination of three markers for detecting lung cancer in different histological types. Tables F and G provide the raw data from which the graphs of Figures 14 and 15A-BB were generated. This data illustrates the strong correlation of expression of CEA, CK7, CK19, PVA, SCCA1.2, SFTPB, and TACSTD1 markers, alone or in combination, in sentinel lymph nodes with the presence of malignant cells arising from a lung cancer in the sentinel lymph node.

**Table F. Single Marker Prediction Characteristics for Lung Cancer**

| | Observed Data | | | Cross Validation Estimates* | | | |
|---|---|---|---|---|---|---|---|
| | Sensitivity | Specificity | Classification | Sensitivity | Specificity | Classification | Bias* |
| CEA | 1.0 | .952 | .976 | .952 | .905 | .928 | .048 |
| CK7 | .810 | .952 | .881 | .762 | .905 | .833 | .048 |
| CK19 | 1.0 | 1.0 | 1.0 | .952 | .952 | .952 | .048 |
| LUNX | 1.0 | .857 | .929 | .952 | .857 | .905 | .024 |
| PVA | .667 | 1.0 | .833 | .619 | .952 | .786 | .048 |
| SCCA1.2 | .810 | .667 | .738 | .524 | .524 | .524 | .214 |
| SFTPB | .619 | .952 | .786 | .571 | .762 | .667 | .119 |
| TACSTD1 | 1.0 | 1.0 | 1.0 | 1.0 | 1.0 | 1.0 | 0.0 |

| | | | | | | | |
|---|---|---|---|---|---|---|---|
| *Leave-one-out cross validation ** classification Bias = difference in classification accuracies between observed data and cross validation estimates. This estimates the optimism in using the original data to characterize the decision rule. | | | | | | | |

**Table G. Two Marker Prediction Characteristics for Lung Cancer**

| | Observed Data | | | Parametric Bootstrap Estimates* | | | Bias** |
|---|---|---|---|---|---|---|---|
| | Sensitivity | Specificity | Classification Accuracy | Sensitivity | Specificity | Classification Accuracy | |
| CEA + CK7 | 1.0 | .952 | .976 | .952 | .952 | .952 | .024 |
| CEA + CK19 | 1.0 | .952 | .976 | 1.0 | .952 | .976 | 0 |
| CEA + LUNX | .952 | .952 | .952 | .952 | .952 | .952 | 0 |
| CEA + PVA | .952 | 1.0 | .976 | .952 | 1.0 | .976 | 0 |
| CEA + SCCA1.2 | .952 | 1.0 | .976 | .952 | 1.0 | .976 | 0 |
| CEA + SFTPB | .905 | 1.0 | .952 | .905 | 1.0 | .952 | 0 |
| CEA + TACSTD1 | 1.0 | 1.0 | 1.0 | 1.0 | 1.0 | 1.0 | 0 |
| CK7 + CK19 | 1.0 | .952 | .976 | 1.0 | .952 | .976 | 0 |
| CK7 + LUNX | .810 | .952 | .881 | .810 | .952 | .881 | 0 |
| CK7 + PVA | .905 | .952 | .929 | .905 | .952 | .929 | 0 |
| CK7 + SCCA1.2 | .952 | .952 | .952 | .952 | .952 | .952 | 0 |
| CK7 + SFTPB | .810 | .952 | .952 | .810 | .952 | .881 | .071 |
| CK7 + TACSTD1 | 1.0 | 1.0 | 1.0 | 1.0 | 1.0 | 1.0 | 0 |
| CK19 + LUNX | 1.0 | .952 | .976 | 1.0 | .952 | .976 | 0 |
| CK19 +PVA | 1.0 | .952 | .976 | 1.0 | .952 | .976 | 0 |
| CK19 + SCCA1.2 | 1.0 | .952 | .946 | 1.0 | .952 | .976 | 0 |
| CK19 + SFTPB | 1.0 | 1.0 | 1.0 | 1.0 | .952 | .976 | .024 |
| CK19+TACSTD1 | 1.0 | 1.0 | 1.0 | 1.0 | 1.0 | 1.0 | 0 |
| LUNX+PVA | .857 | .905 | .881 | .762 | .905 | .833 | .048 |
| LUNX + SCCA1.2 | .905 | .905 | .905 | .857 | .905 | .881 | .024 |
| LUNX + SFTPB | .762 | .905 | .833 | .714 | .905 | .810 | .023 |
| LUNX + TACSTD1 | 1.0 | 1.0 | 1.0 | 1.0 | 1.0 | 1.0 | 0 |
| PVA+ SCCA1.2 | .571 | .857 | .714 | .476 | .810 | .643 | .071 |
| PVA + SFTPB | .762 | 1.0 | .881 | .762 | 1.0 | .881 | 0 |
| PVA+TACSTD1 | 1.0 | 1.0 | 1.0 | 1.0 | 1.0 | 1.0 | 0 |
| SCCA1.2 + SFTPB | .857 | .952 | .905 | .810 | .905 | .857 | .048 |
| SCCA1.2+TACSTD1 | 1.0 | 1.0 | 1.0 | 1.0 | 1.0 | 1.0 | 0 |
| SFTPB + TACSTD1 | 1.0 | 1.0 | 1.0 | 1.0 | 1.0 | 1.0 | 0 |

| | | | | | | | |
|---|---|---|---|---|---|---|---|
| * Leave-one-out cross validation ** Classification Bias = average difference in classification accuracies of the bootstrap decision rule applied to original data and the bootstrap decision rule applied to the bootstrap data. This estimates the optimism in using the original data to characterize the decision rule. | | | | | | | |

### Example 4 - Follow-on study - Breast cancer

### Materials and Methods

As outlined above in Example 2, an extensive literature and database survey identified potential mRNA markers for detection of lymph node metastases in breast cancer. A primary screen analyzed the relative expression of 43 potential markers in 6 primary breast tumors and 10 benign lymph nodes obtained from patients without cancer. Six markers showed good characteristics for lymph node metastasis detection and entered a secondary screening phase where expression was analyzed in 25 primary tumors, 27 histologically positive lymph nodes and 21 benign lymph nodes from patients without cancer (73 independent patients). Based on the classification characteristics, 4 markers were selected for an external validation study of 90 SLN from independent patients with breast cancer using a rapid, multiplex real-time PCR assay. Finally, 9 histologically negative and 9 histologically positive lymph nodes were analyzed using a completely automated and rapid RNA isolation and real-time PCR assay on the GeneXpert®.

**Source of Tissues.** Tissues for the marker screening and the GeneXpert® study were obtained from tissue banks at the University of Pittsburgh Medical Center and SLN for the marker validation study were obtained from the Minimally Invasive Molecular Staging of Breast Cancer Trial (MIMS) initiated at the Medical University of South Carolina.

**Tissue Preparation and Histologic Analysis.** All tissues were snap-frozen in liquid nitrogen and stored at -80°C until use, at which time they were embedded in optimal cutting temperature (OCT) compound for frozen sectioning on a cryostat. For the marker screening and GeneXpert® studies, forty 5-micron sections were cut for RNA isolation. Additional sections from the beginning, middle and end of the sections for RNA isolation were cut for H&E and IHC analysis. All three H&E slides from each specimen underwent pathological review by two pathologists. All unstained slides were stored at -20°C and used for IHC evaluation (with the AE1/AE3 pancytokeratin antibody cocktail) as needed to confirm the H&E histology.

For the validation study, 115 chronologically-obtained SLN specimens from individual patients were identified. Five-micron serial sections were cut from each tissue, and the initial and final two tissue sections were mounted on slides for histological analysis with H&E staining and pancytokeratin IHC. The intervening sections were distributed 4:1:4:1:4 etc., such that four sections were immediately placed in chaotropic lysis buffer for RNA isolation and every fifth section was mounted on a slide for histology review. The total number of sections cut was dependent on size of the SLN (range 50-60). All specimens were reviewed to confirm adequate preservation of histology for pathologic analysis resulting in the exclusion of 25 specimens. For the remaining 90 SLN, sections from three levels (beginning, middle and end) underwent pathologic review with both H&E and IHC staining, and remaining slides were reviewed as needed.

All specimens were independently evaluated by two pathologists with extensive experience interpreting breast cancer specimens. The pathologists determined the presence of tumor, the percentage of tumor, and the presence of any contaminating tissues (-e.g.. normal breast tissue). Discordantly interpreted specimens were noted, and then reviewed simultaneously and consensus made.

RNA Isolation. For the screening and validation studies, RNA was isolated using the RNeasy minikit (Qiagen, Valencia, CA) as described by the manufacturer. The only modification was that the volume of lysis reagent was doubled and loaded on the column in two steps. All RNA's were DNAse treated using the DNA-free Kit from Ambion.

**Quantitative RT-PCR Analysis.** For the marker screening study, cDNA was synthesized using random hexamers. Quantitative real-time PCR was performed on the ABI Prism 7700 Sequence Detection instrument and expression of each marker gene was measured relative to the endogenous control gene β-glucuronidase using ΔCt calculations. To save cost, the primary screen was performed using quantification with SYBR green. In the secondary screen, 5' nuclease hybridization probes were used to increase assay specificity. All assays were designed using the ABI Primer Express Version 2.0 software and where possible, amplicons spanned exon junctions to provide cDNA specificity. Negative controls were included in each PCR plate. A mixture of the Universal Human Reference RNA (Stratagene, La Jolla, CA) and RNAs from human placenta, thyroid, heart, colon, PCI13 cell line and SKBR3 cell line served as a universal positive expression control for all the genes in the marker screening process.

Analysis of the four genes in the marker validation study was performed using rapid, multiplex (endogenous control gene and target gene) QRT-PCR on the Cepheid SmartCycler™ (Cepheid, Sunnyvale, CA). RNA input for each lymph node sample was 50-200 ng per QRT-PCR reaction and all reactions were performed in duplicate. Each reaction incorporated an internal positive control (IPC) oligonucleotide to demonstrate adequate assay sensitivity in the case of negative results. Gene specific reverse transcription primer sequences and PCR primer and probe sequences are shown in Table B.

**GeneXpert Analysis.** Twenty-four, 5-µm sections of OCT embedded tissue were sectioned into 800 µl of GeneXpert^{®} lysis buffer (Cepheid, Sunnyvale, CA). The lysis buffer was filtered through a 0.22-µm syringe filter (Osmonics Inc, West borough, MA), and loaded into a GeneXpert^{®} cartridge. The automated processes of RNA isolation, reverse transcription, and QRT-PCR on the GeneXpert®.

**Statistical Analyses.** The characteristics used to evaluate markers were sensitivity, specificity, classification accuracy and negative and positive predictive values. The evaluation included characterizing the distributions of the markers and testing the fit of the data to the log-normal distribution. For individual markers, a cutoff value was determined that maximized the classification accuracy. In cases where classification accuracy was 100%, the cutoff was set at the midpoint between the highest expressing benign node and the lowest expressing histologically positive node. Markers were also evaluated in paired combinations and a linear prediction rule was generated for each pair. The rule was equivalent to the linear predictor that equalized the fitted probabilities above and below the linear boundary. That is, points on the boundary line had a predicted probability midway between the numeric scores assigned to positive and negative nodes.

Properties of single and paired marker prediction rules were also investigated by examining the distributional properties of the expression levels and by applying parametric bootstrap validation. Data were simulated from the lognormal and bivariate lognormal distributions using moment estimators for mean, variance and correlation between marker pairs. Five hundred parametric samples of the original data were obtained and the prediction for each bootstrap sample was applied to the original data. Using Efron's improved bootstrap for prediction error (Efron B, TR. An Introduction to the Bootstrap. Boca Raton: Chapman and Hall, 1993: 247-252), the difference between the observed classification accuracy and the average bootstrap classification accuracy was used to estimate the optimism in the resubstitution prediction rules. The single marker and double marker decision rules were then applied to data from the marker validation study and classification characteristics were calculated.

Prediction characteristics of marker combinations were also determined by generating equal probability contours. In this method, the joint distributions of marker pairs were assumed to follow a bivariate log-normal distribution. From estimates of the means, variances and covariances of benign nodes, equal probability contours were constructed around the estimated mean values obtained for relative level of expression in benign lymph nodes. Observed values were then plotted against these equal probability ellipsoids and compared to contours for the more extreme quantiles, including the 95^{th}, 99^{th} and 99.9^{th} percentiles. This method of analyzing the data attaches a value to each point that is the approximate probability that the plotted node is benign.

### Results

**Primary Marker Screen.** Median relative expression in primary tumors and in benign lymph nodes was calculated for all 43 potential markers included in the primary screen (Table H).

| **Table H - Relative expression in primary tumor and benign lymph node from primary screening** | | | | | | |
|---|---|---|---|---|---|---|
| **Gene** | **Accession Number** | **Median Tumor** | **Median Benign Node** | **Highest Benign Node** | **Lowest Tumor/Highest Benign Node** | **Median Tumor/Highest Benign Node** |
| TACSTD1 | NM_004616 | 56.797758 | 0.009753 | 0.049549 | 643.6 | 1146.3 |
| CK19 | NM_002276 | 34.646797 | 0.003691 | 0.018136 | 1086.1 | 1910.4 |
| CK7 | NM_005556 | 14.725973 | 0.000953 | 0.003162 | 22.2 | 4657.3 |
| CK18 | NM_000224 | 6.956115 | 0.033609 | 0.073557 | 18.1 | 94.6 |
| MMP7 | NM_002423 | 0.626631 | 0.009453 | 0.099098 | 1.6 | 6.3 |
| MGB1 | NM_002411 | 0.283272 | 0.000034 | 0.000207 | 10.1 | 1365.5 |
| Survivin | NM_003317 | 0.186357 | 0.022251 | 0.113834 | 0.1 | 1.6 |
| PIP | NM_002652 | 0.117351 | 0.000519 | 0.001258 | 1.0 | 93.3 |
| MGB2 | NM_002407 | 0.106771 | _*^{c}* | _*^{c}* | ∞ | ∞ |
| c-MET | NM_000245 | 0.061745 | 0.020617 | 0.039692 | 0.3 | 1.6 |
| PTHrP | NM_002820 | 0.024784 | 0.001186 | 0.004425 | 2.5 | 5.6 |
| NIS | NM_000453 | 0.015681 | 0.003933 | 0.016232 | 0.02 | 1.0 |
| TM4SF3 | NM_004616 | 0.013634 | 0.025295 | 0.136787 | 0.01 | 0.1 |
| BHCG | NM_000737 | 0.007882 | 0.001893 | 0.009005 | 0.4 | 0.9 |
| CEA | NM_004363 | 0.007112 | 0.000175 | 0.000270 | 1.3 | 26.3 |
| SCCA1.2 | NM_006919 | 0.005290 | 0.000014 | 0.015571 | 0.04 | 0.3 |
| MAGEA8 | NM_005364 | 0.004843 | _*^{c}* | _*^{c}* | ∞ | ∞ |
| Villinl | NM_007127 | 0.003967 | 0.000288 | 0.000433 | 0.8 | 9.2 |
| KRTHB1 | NM_002281 | 0.002590 | _*^{b}* | 0.000165 | 4.2 | 15.7 |
| TITF1 | NM_007127 | 0.001900 | 0.001320 | 0.007625 | 0.03 | 0.2 |
| HTERT | NM_003219 | 0.001455 | 0.012648 | 0.026645 | 0.01 | 0.1 |
| ITGB4 | NM_000213 | 0.000799 | 0.000038 | 0.000063 | 3.2 | 12.6 |
| STX | NM_002354 | 0.000728 | 0.000030 | 0.000194 | 0.8 | 3.7 |
| LDHC | NM_017448 | 0.000487 | _*^{b}* | 0.016402 | 0.00006 | 0.03 |
| BAGE | NM_001187 | 0.000445 | _*^{b}* | 0.000152 | 0.007 | 2.9 |
| CTAG1 | NM_001327 | 0.000416 | _*^{b}* | 0.002036 | 0.0005 | 0.2 |
| NTS | NM_006183 | 0.000305 | 0.532185 | 2.321408 | 0.0 | 0.001 |
| MAGEA2 | NM_005361 | 0.000183 | _*^{b}* | 0.000279 | 0.004 | 0.7 |
| CK20 | NM_019010 | 0.000161 | _*^{c}* | _*^{c}* | ∞ | ∞ |
| GAGE1 | NM_001468 | 0.000144 | _*^{b}* | 0.000703 | 0.001 | 0.2 |
| SSX2 | NM_006011 | 0.000136 | _*^{c}* | _*^{c}* | ∞ | ∞ |
| MAGEA3 | NM_005362 | 0.000116 | 0.000328 | 0.001271 | 0.001 | 0.1 |
| SSXu*^{a}* | NM_001169 | 0.000061 | _*^{c}* | _*^{c}* | ∞ | ∞ |
| BRDT | NM_001726 | 0.000037 | 0.000278 | 0.000350 | 0.003 | 0.1 |
| SGY-1 | NM_014419 | 0.000024 | 0.000134 | 0.000605 | 0.002 | 0.04 |
| GAGEu*^{a}* | | 0.000023 | 0.000010 | 0.000126 | 0.1 | 0.2 |
| MAGEA12 | NM_005367 | 0.000022 | 0.000103 | 0.000404 | 0.002 | 0.1 |
| MAGEA1 | NM_004988 | 0.000017 | _*^{c}* | _*^{c}* | ∞ | ∞ |
| MAGEA4 | NM_002362 | 0.000011 | _*^{c}* | _*^{c}* | ∞ | ∞ |
| CK14 | NM_000526 | _*^{c}* | _*^{c}* | _*^{c}* | ∞ | ∞ |
| LUNX | NM_130852 | _*^{b}* | _*^{b}* | _*^{b}* | ∞ | ∞ |
| MAGEA10 | NM_021048 | _*^{c}* | _*^{c}* | _^{c} | ∞ | ∞ |
| TYR | NM_000372 | _*^{b}* | _*^{c}* | _*^{c}* | ∞ | ∞ |

In addition, the ratio was calculated between the median expression in tumors and the highest expressing benign node and between the lowest expressing tumor and the highest expressing benign node. When using median expression in the tumors as the numerator, four genes, TACSTD1, cytokeratin 7 (CK7), cytokeratin 19 (CK19), and mammoglobin 1 (MGB1) stood out as having tumor/benign node ratios greater than 1000. Thus, these 4 markers were selected for further evaluation. Mammoglobin 2 (MGB2) and prolactin inducible protein (PIP) were also selected based on the primary screen data as well as previously published data regarding these markers (Mitas M, Mikhitarian K, Walters C, Baron PL, Elliott BM, Brothers TE et al. Quantitative real-time RT-PCR detection of breast cancer micrometastasis using a multigene marker panel. Int J Cancer 2001; 93(2):162-171). The other 37 markers were excluded from further evaluation.

**Secondary Marker Screen.** Histologic evaluation of the 25 primary breast cancer specimens used in the secondary screen revealed a median tumor percentage of 75% (range of 5-95%). The median tumor percentage in the 27 histologically positive nodes was 80% (range of 5-95%). The relative expression of the 6 markers included in the secondary screen in breast tumors, positive lymph nodes, and benign lymph nodes are shown in Figure 12. The classification characteristics of each marker (compared with pathology review) are summarized in Table I.

| **Table I - Single or two marker prediction characteristics in secondary screening** | | | | | | | |
|---|---|---|---|---|---|---|---|
| | **Observed Data** | | | **Parametric Bootstrap Estimates*** | | | |
| **Marker** | **Sensitivity** | **Specificity** | **Classification Accuracy** | **Sensitivity** | **Specificity** | **Classification Accuracy** | **Classification Bias**** |
| CK7 | .889 | .952 | .917 | .828 | .909 | .863 | .054 |
| CK19 | 1.0 | .952 | .979 | .997 | .891 | .951 | .028 |
| MGB1 | .926 | .857 | .896 | .903 | .748 | .836 | .060 |
| MGB2 | .963 | .905 | .938 | .943 | .834 | .895 | .043 |
| PIP | .852 | .952 | .896 | .814 | .892 | .848 | .048 |
| TACSTD1 | 1.0 | 1.0 | 1.0 | .999 | .956 | .980 | .020 |
| CK7 + CK19 | 1.0 | .905 | .958 | .993 | .868 | .938 | .020 |
| CK7 + MGB1 | .963 | 1.0 | .979 | .954 | 1.0 | .977 | .002 |
| CK7 + MGB2 | 1.0 | 1.0 | 1.0 | 1.0 | 1.0 | 1.0 | .000 |
| CK7 + PIP | .963 | 1.0 | .979 | .963 | 1.0 | .979 | .000 |
| CK7 + TACSTD1 | .963 | 1.0 | .979 | .928 | 1.0 | .959 | .020 |
| CK19 + MGB1 | 1.0 | 1.0 | 1.0 | .996 | 1.0 | 1.0 | .000 |
| CK19 + MGB2 | .963 | 1.0 | .979 | .945 | .979 | .975 | .004 |
| CK19 + PIP | .926 | 1.0 | .958 | .900 | 1.0 | .951 | .007 |
| CK19 + TACSTD1 | .963 | 1.0 | .979 | .928 | 1.0 | .960 | .019 |
| MGB1 + MGB2 | .889 | .952 | .917 | .853 | .925 | .885 | .032 |
| MGB1 + PIP | .963 | .905 | .938 | .963 | .876 | .934 | .004 |
| MGB1 + TACSTD1 | .963 | 1.0 | .979 | .942 | 1.0 | .967 | .012 |
| MGB2 + PIP | .926 | 1.0 | .958 | .915 | 1.0 | .953 | .005 |
| MGB2 + TACSTD1 | .963 | 1.0 | .979 | .930 | 1.0 | .961 | .018 |
| PIP + TACSTD1 | .963 | 1.0 | .979 | .929 | 1.0 | .960 | .017 |

The observed classification accuracies ranged from 89.6% (MGB1 and PIP) to 100% (TACSTD1). Parametric bootstrap analysis of this data is also shown in Table I and the estimates of classification bias ranged from 2% (TACSTD1) to 6% (MGB1). Thus, the relative expression level cut-offs established for each individual marker in the screening set should accurately characterize subsequently analyzed lymph nodes.

We also examined all possible combinations of marker pairs to determine if an assay that evaluates more than one marker produces a more robust lymph node characterization. The relative expression of each possible marker pairing was analyzed using a linear decision rule that optimized characterization accuracy and these decision rules were again internally validated using a parametric bootstrap analysis. This data is depicted in Figure 17A-17H and summarized in Table I. Eleven of the 15 combinations provided 100% classification accuracy in the observed data but only two combinations retained 100% predicted accuracy in the bootstrap analysis. In general, the use of a pair of markers resulted in a reduction in classification bias (0-3.2%) confirming that a 2-marker assay improved assay classification confidence.

Since linear classification rules are not necessarily the best method for lymph node classification in the marker combination analysis, a novel classification method was developed based on the observed distribution of expression levels for each marker in a given pair. Equal probability contours were calculated around the mean values obtained for relative expression in benign lymph nodes (Figure 17E-17H). This method of analysis demonstrates that the distribution of relative expression values obtained from benign lymph nodes impacts the confidence for classifying a positive lymph node. While CK19/MGB1 provided the best classification based on a linear prediction rule, the probability contour plot clearly shows that the wide distribution of expression for both of these markers in benign nodes negatively impacts the confidence with which a positive node can be identified. By this analysis, the combinations of TACSTD1/PIP, CK19/TACSTD1, TACSTD1/MGB1 and TACSTD1/MGB2 provide the best classification with all positive nodes correctly identified with probabilities >0.99 and in most cases >0.999. For all four of these combinations, all benign nodes fell within the 0.99 probability contour and all but one was within the 0.95 probability contour. Therefore, in the screening data, four marker combinations were capable of providing 100% sensitivity with >99% specificity.

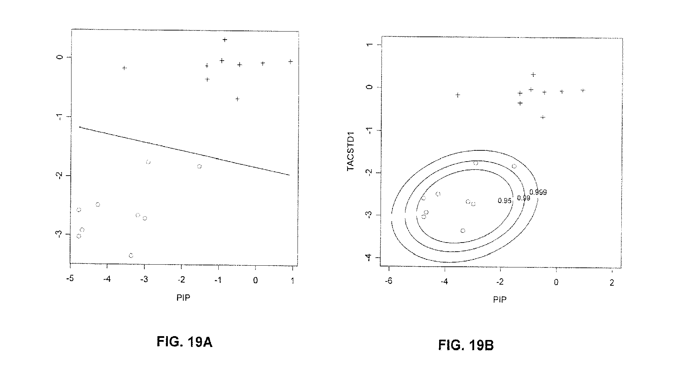

**Validation of QRT-PCR classification in a Rapid, Multiplex format.** To externally validate the classification accuracy of selected markers tested in the secondary screen, an independent, validation set of 90 breast cancer sentinel lymph nodes was prospectively analyzed (Figures 19A and 19B). Furthermore, to demonstrate the potential for intraoperative analysis, this study was performed on the SmartCycler® instrument (Cepheid) using rapid, multiplex QRT-PCR. Subtle differences in calculated relative expression values were observed (data not shown) from this change in thermocycler platform, but in an effort to indirectly evaluate the robustness of the QRT-PCR analysis, the classification algorithms from the secondary screen were applied to the validation set data without any correction factors.

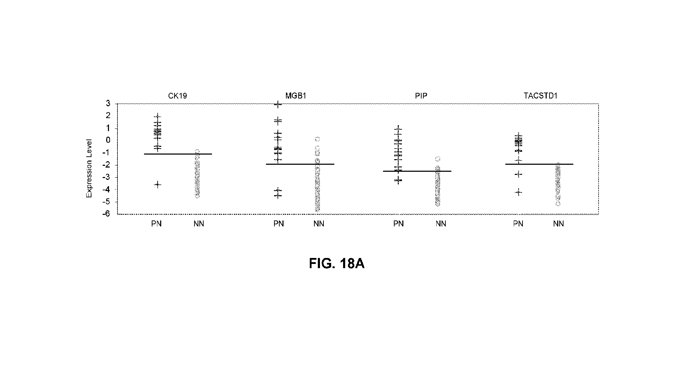

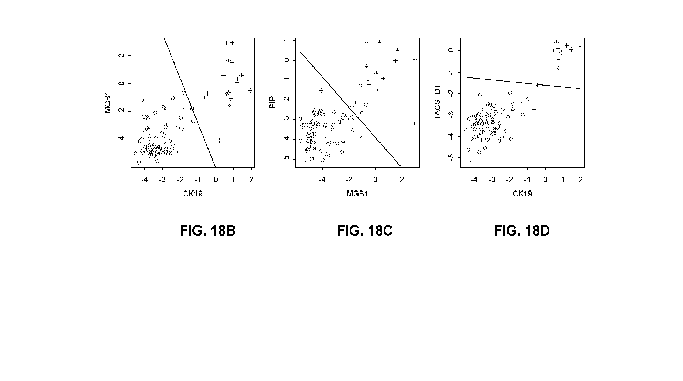

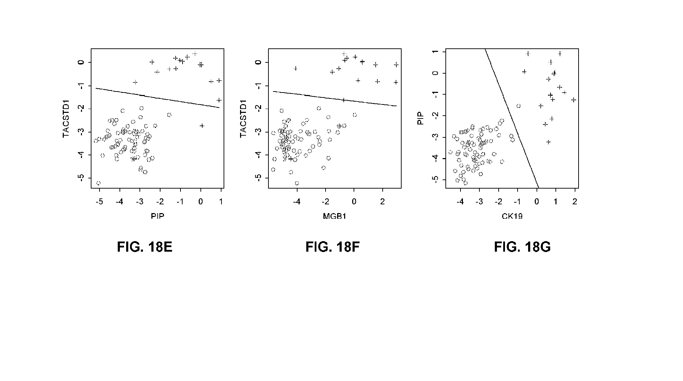

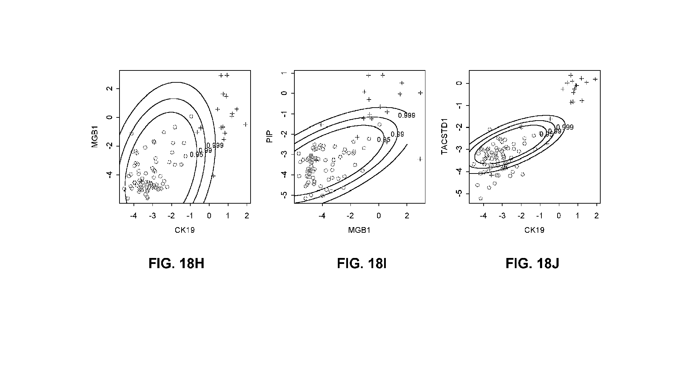

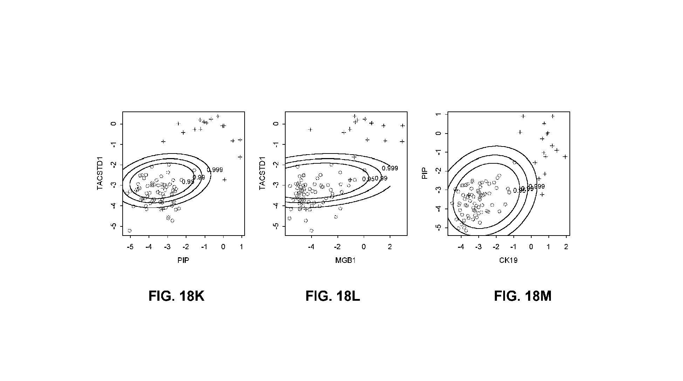

Pathologic review identified 73 negative SLN's and 17 SLN's positive for metastasis, with a median tumor percentage in the positive lymph nodes of 60% (range 5% - 95%). The relative expression data for each of the 4 selected markers, and marker combinations, is shown in Figure18A-M, and prospective classification accuracy for individual markers and all potential marker pairs is reported in Table J.

**Table J Validation set results. Prospective classification characteristics of QRT-PCR assays using single or paired markers**

| **Marker/Combination)** | **Sensitivity** | **Specificity** | **Accuracy** | **NPV*** | **PPV**** |
|---|---|---|---|---|---|
| | | | | | |
| PIP | .882 | .959 | .944 | .972 | .833 |
| MGB1 | .882 | .890 | .889 | .970 | .652 |
| TACSTD1 | .882 | 1.0 | .978 | .973 | 1.0 |
| CK19 | .941 | .986 | .941 | .978 | .986 |
| | | | | | |
| PIP + MGB1 | .882 | .944 | .933 | .971 | .789 |
| PIP + TACSTD1 | .882 | 1.0 | .978 | .973 | 1.0 |
| PIP + CK19 | .941 | .986 | .978 | .986 | .941 |
| MGB1 + TACSTD1 | .823 | 1.0 | .966 | .960 | 1.0 |
| MGB1 + CK19 | .941 | .986 | .978 | .986 | .941 |
| TACSTD1 + CK19 | .823 | 1.0 | .966 | .960 | 1.0 |

| | | | | | |
|---|---|---|---|---|---|
| *NPV = negative predictive value; **PPV = positive predictive value. | | | | | |

When cut-off values (individual markers) or linear prediction rules (marker combinations) from the secondary screen were applied to the validation set data, overall classification accuracy ranged from 89% (MGB1 alone) to 98% (TACSTD1 alone, TACSTD1/PIP, PIP/CK19 and MGB1/CK19). When probability contours from the secondary screen were applied, several marker combinations identified 16/17 (94%) of positive nodes with > 99.9% probability while all negative nodes fell within the 99% probability contour. One histologically positive sample was characterized as negative with >95% probability by analysis with all 4 markers. In a post-analysis review, this specimen was found to have been discordantly interpreted by the two pathologists. A concurrent opinion had been reached, based on a very small focus of tumor in the first 2 serial sections that was not present in the remaining 8 slides. Thus, our finding that this specimen was consistently classified as negative by QRT-PCR may represent sampling error.

From our data, we conclude there are a number of mRNA markers and marker combinations capable of accurately detecting metastatic breast cancer in lymph nodes. However, there are at least 3 pseudogenes for CK19 within the human genome that lack intronic sequence. Thus, an mRNA-specific primer set cannot be designed for CK19, and failure of DNAse treatment to completely digest contaminating genomic DNA within the sample could produce a false positive result. Thus, the combination that produces the highest accuracy without other potentially negative attributes is the marker pair of TACSTD1 and PIP.

**Automated Lymph Node Analysis with the GeneXpert^{®}.** Eighteen lymph node specimens from individual patients were evaluated with fully automated, QRT-PCR assays for the markers TACSTD 1 and PIP (Figures 19A and 19B). Histologic review confirmed that this set consisted of 9 positive (60-95% tumor) and 9 negative lymph nodes. When prospectively analyzed by either a linear decision rule or equal probability contour analysis using decision rules based on data from the secondary screen set, the multiplex GeneXpert® assay accurately (100%) characterized all 18 specimens within 35 minutes per assay. We conclude that a fully automated, rapid QRT-PCR assay accurately characterizes lymph nodes for the presence of metastatic breast cancer.

The above-described methods are seen to provide exceptional accuracy detecting metastatic disease within the SLN of breast cancer patients using a 2-marker QRT-PCR assay compared to the current methods of complete SLN analysis including histological and immunohistochemical review. Also demonstrated is the accurate classification of the lymph node specimens obtained when the assay was fully automated using the GeneXpert® instrument. Thus, this assay surpasses the accuracy of current frozen section analysis of SLNB specimens, and is potentially superior to complete histological and IHC analysis in that: 1) it is fully automated, reducing the potential for human error, 2) it uses objective criteria, removing subjective analysis and improving standardization, and 3) it is completed in less than 35 minutes, facilitating intraoperative use and reducing anxious apprehension for the patient.

Previous studies have aimed to determine if RT-PCR analysis of lymph nodes is more sensitive than IHC and thus capable of further improving the clinical staging of breast cancer patients. The present study differs from those studies in that the present aim was not to determine if QRT-PCR identified metastatic disease in definitively analyzed, histologically negative SLN, but rather to surpass current methods of analysis with regards to timeliness, reproducible objectivity, and automation. However, based on the published literature regarding sensitivity of QRT-PCR analyses and the ability of this automated assay to improve sampling by evaluating a larger percentage of the LN (current SLNB analysis examines less than 1.5% of the specimen), it is believed that this assay may prove to be capable of surpassing current techniques in this regard.

This assay ultimately may prove to be superior to conventional histological analysis because of the objective nature of the test, but this benefit is implied and has not yet been scientifically proven. The accurate histological analysis of lymph nodes for micrometastatic disease is challenging under ideal conditions, by nature subjective, and the interpretation of microscopic foci of tumor cells has eclipsed clinical outcome data. The AJCC Cancer Staging Manual, 6th edition has established definitions to facilitate consistency in interpretation of these materials, yet these definitions make further demands on the pathologist's subjective interpretation of the lymph node. In the only published study examining this problem, Roberts, *et al.* found that when 10 pathologists evaluated 25 cases of breast cancer SLNB specimens, only 12% of the cases were correctly classified by all the pathologists, and 80% of the IHC-positive cases had at least one pathologist incorrectly characterize the case (Roberts CA, Beitsch PD, Litz CE, Hilton DS, Ewing GE, Clifford E et al. Interpretive disparity among pathologists in breast sentinel lymph node evaluation. Am J Surg 2003; 186(4):324-329). In contrast, as demonstrate herein and separately, the fully automated QRT-PCR assay is robust and objective. Thus, a reproducible, fully automated, objective analysis of SLNs has the potential to be superior to current methods of analysis, and a multi-center, prospective trial designed to make this comparison is currently in development.

In summary, it has been shown that a 2-marker, QRT-PCR assay that is fully automated and completed in under 35 minutes can accurately characterize lymph nodes for the presence of metastatic breast cancer. This assay is clearly superior to current methods of intraoperative analysis and is as accurate as current methods of complete histological analysis including immunohistochemical analysis. Theoretical advantages to such an assay include improved standardization across varying healthcare environments, increased sampling of the lymph node, and reduced human error.

## Claims

1. A method of identifying expression of markers indicative of the presence of breast cancer cells in a lymph node of a patient, comprising determining if both a first mRNA species specific to TACSTD1 and a second mRNA species specific to PIP are overabundant in an RNA sample prepared from the lymph node;
the overabundance of both the first and second mRNA species being indicative of the presence of displaced breast cancer cells in the lymph node.

2. The method of claim 1, comprising quantifying levels of the mRNA species in the RNA sample and determining if the first and second mRNA species are overabundant in the RNA sample.

3. The method of claim 1, wherein a nucleic acid amplification assay is used to determine if the first and second mRNA species are overabundant in the RNA sample.

4. The method of claim 3, wherein the nucleic acid amplification assay is a PCR assay or an isothermic amplification assay.

5. The method of claim 4, wherein the nucleic acid amplification assay is an assay selected from the group consisting of RT-PCR, QRT-PCR, rolling circle amplification and nucleic acid sequences-based amplification assays.

6. The method of claim 5, wherein the assay is an RT-PCR assay.

7. The method of claim 6, wherein the RT-PCR assay uses one or more primer pairs specific to PIP or TACSTD1.

8. The method according to claim 7, wherein the primer pairs consist essentially of at least about ten contiguous nucleic acids of
PIP primers SEQ ID No 5, bases 333 to 357 and SEQ ID No 18; or
TACSTD1 primers SEQ ID No 10, bases 348 to 368 and SEQ ID No 22.

9. The method of claim 3, wherein the assay is a multiplex assay.
